# EUROPEAN PATENT SPECIFICATION

(11) **EP 4 359 127 B1**
(45) Date of publication and mention of the grant of the patent: **21.05.2025**
(21) Application number: 22731803.7
(22) Date of filing: 25.05.2022
(51) Int. Cl.: B01L 3/00, G01N 33/24

(54) **MICROFLUIDIC MANIFOLD AND METHOD FOR DETERMINATION OF ANALYTE CONCENTRATION FROM TEMPERATURE-COMPENSATED ABSORBANCE MEASUREMENTS**
MIKROFLUIDISCHER VERTEILER UND VERFAHREN ZUR BESTIMMUNG DER ANALYTKONZENTRATION AUS TEMPERATURKOMPENSIERTEN ABSORPTIONSMESSUNGEN
COLLECTEUR MICROFLUIDIQUE ET PROCÉDÉ DE DÉTERMINATION DE CONCENTRATION D'ANALYTE À PARTIR DE MESURES D'ABSORBANCE À COMPENSATION DE TEMPÉRATURE

(30) Priority: 22.06.2021 US 202163213319 P
(43) Date of publication of application: 01.05.2024
(73) Proprietor: Precision Planting LLC, Tremont, IL 61568 (US)
(72) Inventor: SWANSON, Todd, TREMONT, Illinois 61568 (US); VACCARI, Adam, TREMONT, Illinois 61568 (US); SCHAEFER, Timothy, Tremont, Illinois 61568 (US); LITWILLER, Riley, TREMONT, Illinois 61568 (US)
(74) Representative: AGCO Intellectual Property Department
(86) International application number: PCT/IB2022/054922
(87) International publication number: WO 2022/269388

(56) References cited:
- US-A1- 2012 051 390
- US-A1- 2019 176 149

## Description

### BACKGROUND

The present invention relates generally to agricultural sampling and analysis, and more particularly to a fully automated system for performing soil and other types of agricultural related sampling and chemical property analysis.

Periodic soil testing is an important aspect of the agricultural arts. Test results provide valuable information on the chemical makeup of the soil such as plant-available nutrients and other important properties (e.g. levels of nitrogen, magnesium, phosphorous, potassium, pH, etc.) so that various amendments may be added to the soil to maximize the quality and quantity of crop production.

In some existing soil sampling processes, collected samples are dried, ground, water is added, and then filtered to obtain a soil slurry suitable for analysis. Extractant is added to the slurry to pull out plant available nutrients. The slurry is then filtered to produce a clear solution or supernatant which is mixed with a chemical reagent for further analysis.

US 2019/176149 A1 discloses an apparatus for performing a field assay of an analyte with a microfluid cartridge and a reader. The microfluidic cartridge has a closed microfluidic circuit for mixing and recirculating a fluid. The reader is selectively communicated with the microfluidic cartridge. The first manifold is configured to distribute fluid uniformly to a plurality of channels of a surface acoustic wave (SAW) detector. A second manifold coupled to a return line is configured to remove the fluid from the plurality of channels of the SAW detector.

Improvements in testing soil, vegetation, and manure are desired.

### BRIEF SUMMARY

The present invention provides an automated computer-controlled sampling system and related methods for collecting, processing, and analyzing soil samples for various chemical properties such as plant available nutrients (hereafter referred to as a "soil sampling system"). The sampling system allows multiple samples to be processed and analyzed for different analytes (e.g. plant-available nutrients) and/or chemical properties (e.g. pH) in a simultaneous concurrent or semi-concurrent manner, and in relatively continuous and rapid succession. Advantageously, the system can process soil samples in the "as collected" condition without the drying and grinding steps previously described.

The present system generally includes a sample preparation sub-system which receives soil samples collected by a probe collection sub-system and produces a slurry (i.e. mixture of soil, vegetation, and/or manure and water) for further processing and chemical analysis, and a chemical analysis sub-system which receives and processes the prepared slurry samples from the sample preparation sub-system for quantification of the analytes and/or chemical properties of the sample. The described chemical analysis sub-system can be used to analyze soil, vegetation, and/or manure samples.

In one embodiment, the sample preparation system generally includes a mixer-filter apparatus which mixes the collected raw soil sample in the "as sampled" condition (e.g. undried and unground) with water to form a sample slurry. The mixer-filter apparatus then filters the slurry during its extraction from the apparatus for processing in the chemical analysis sub-system. The filter may be separate The chemical analysis sub-system processes the slurry and performs the general functions of extractant and color-changing reagent addition/mixing, centrifugating or filtering the slurry sample via microporous filter to yield a clear supernatant, and finally sensing or analysis for detection of the analytes and/or chemical properties such as via colorimetric analysis. In various embodiments, all or part of the chemical analysis sub-system may be incorporated into one or more microfluidic devices of suitable configuration.

Although the sampling systems (e.g. sample collection, preparation, and processing) may be described herein with respect to processing soil samples which represents one category of use for the disclosed embodiments, it is to be understood that the same systems including the apparatuses and related processes may further be used for processing other types of agricultural related samples including without limitation vegetation/plant, forage, manure, feed, milk, or other types of samples. The embodiments of the invention disclosed herein should therefore be considered broadly as an agricultural sampling system. Accordingly, the present invention is expressly not limited to use with processing and analyzing soil samples alone for chemical properties of interest.

### BRIEF DESCRIPTION OF THE DRAWINGS

The present invention will become more fully understood from the detailed description and the accompanying drawings, wherein like elements are labeled similarly and in which:
FIG. 1 is a schematic flow diagram of an agricultural sampling analysis system according to the present disclosure showing high-level functional aspects of each sub-system of the sampling analysis system;
FIG. 2 is a schematic system diagram of a programmable processor-based central processing unit (CPU) or system controller configured and operable for controlling the microfluidic devices of the microfluidic processing manifold systems and apparatuses disclosed herein;
FIG. 3 is a first perspective view of a multi-layered microfluidic manifold chemical analysis substrate for measuring an analyte in an agricultural slurry sample fluid;
FIG. 4 is a second perspective view thereof;
FIG. 5 is a first exploded perspective view thereof;
FIG. 6 is a second exploded perspective view thereof;
FIG. 7 is an end view thereof;
FIG. 8 is a side view thereof;
FIG. 9 is a first plan view of a first major side thereof;
FIG. 10 is a second plan view of a second major side thereof;
FIG. 11 is a plan view of the liquid layer thereof showing a plurality of microfluidic devices and microchannels which fluidly couple the devices and absorbance measurement device together;
FIG. 12 is a first perspective view of an optical absorbance measurement device of the chemical analysis substrate;
FIG. 13 is a second perspective view thereof;
FIG. 14 is a transverse cross-sectional perspective view thereof;
FIG. 15 is a side transverse cross sectional view thereof;
FIG. 16 is a perspective view of a section of the chemical analysis substrate showing the absorbance measurement device, a temperature sensor, and a portion of a micropump;
FIG. 17 is an enlarged detail taken from FIG. 16;
FIG. 18 is a system block diagram including the programmable controller and the slurry processing and analysis substrates;
FIG. 19 is a graph showing a base calibration curve for determining a concentration of an analyte in an agricultural sample fluid based on measured absorbance; and
FIG. 20 is graph showing a temperature compensation curve is correcting the base calibration curve of FIG. 19 based on measured temperature of the agricultural sample fluid.

All drawings are not necessarily to scale. Components numbered and appearing in one figure but appearing un-numbered in other figures are the same unless expressly noted otherwise. A reference herein to a figure by a whole figure number which may appear in multiple figures bearing the same whole number but with different alphabetical suffixes shall be construed as a general reference to all of those figures unless expressly noted otherwise.

### DETAILED DESCRIPTION

The features and benefits of the invention are illustrated and described herein by reference to exemplary ("example") embodiments. This description of exemplary embodiments is intended to be read in connection with the accompanying drawings, which are to be considered part of the entire written description. Accordingly, the disclosure expressly should not be limited to such exemplary embodiments illustrating some possible non-limiting combination of features that may exist alone or in other combinations of features.

In the description of embodiments disclosed herein, any reference to direction or orientation is merely intended for convenience of description and is not intended in any way to limit the scope of the present invention. Relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise.

As used throughout, any ranges disclosed herein are used as shorthand for describing each and every value that is within the range. Any value within the range can be selected as the terminus of the range.

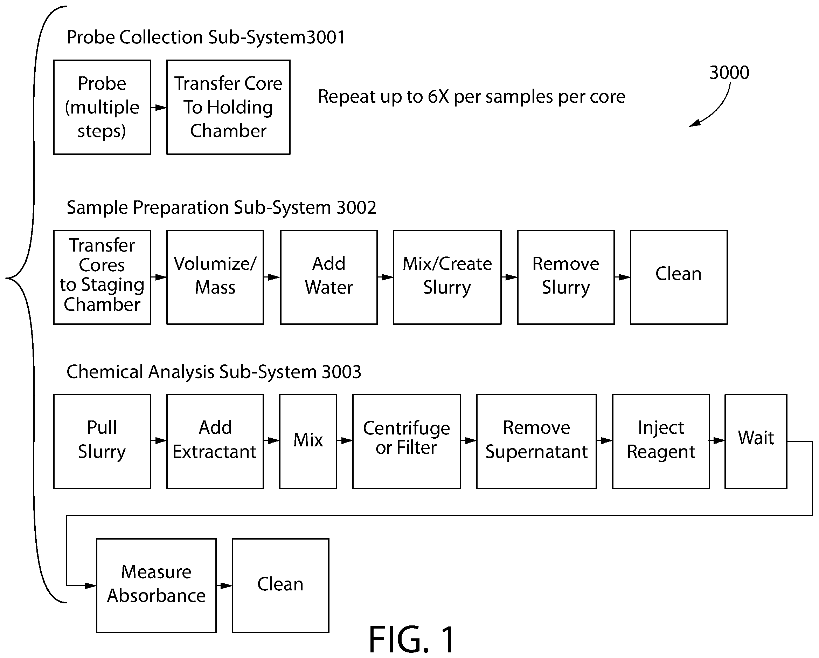

FIG. 1 is a high level schematic diagram flow chart describing the functional aspects of an agricultural sampling system 3000 according to the present disclosure. The system includes multiple sub-systems which operate in concert and sequence. The sub-systems disclosed herein collectively provide complete processing and chemical analysis of soil samples from collection in the agricultural field, sample preparation and processing, and final chemical analysis. The agricultural material sampled may be soil in one embodiment; however, other types of agricultural materials may be processed and analyzed in the same system including without limitation vegetation/plants, crop residues, forage, manure, feed, milk, and other agricultural related materials of interest in the agricultural, livestock, diary or similar arts. In the context of soil sampling for example which is important to crop production and yield, the agricultural sampling system 3000 advantageously allows multiple samples to be processed and chemically analyzed simultaneously for different various plant-available nutrients or other parameters such as for example without limitation pH, BpH (buffer pH), etc.. This information may be used to generate nutrient/parameter maps for the agricultural field to determine the appropriate quantities of soil amendments needed in different regions of the field to maximize overall crop production.

In one embodiment, portions of the agricultural sampling system 3000 may be incorporated onboard a motorized sampling vehicle configured to traverse an agricultural field for collecting and processing soil samples from various zones of the field. This allows a comprehensive nutrient and chemical profile of the field to be accurately generated "on-the-fly" in order to quickly and conveniently identify the needed soil amendments and application amounts necessary in real-time for each zone or region of the field based on quantification of the plant-available nutrient and/or chemical properties in the sample.

The soil sampling system 3000 generally includes a sample probe collection sub-system 3001, a sample preparation sub-system 3002, and a chemical analysis sub-system 3003. The sample collection sub-system 3001 and motorized sampling vehicle are fully described in U.S. Patent Application Publication No. 2018/0124992A1. Sample collection sub-system 3001 generally performs the function of extracting and collecting soil samples from the field. The samples may be in the form of soil plugs or cores. The collected cores are transferred to a holding chamber or vessel for further processing by the sample preparation sub-system 3002. Other sampling systems are described in U.S. Application Nos. 63/191204, filed on 20 May 2021; 17/343434, filed on 09 June 2021; 63/208865, filed on 09 June 2021; 63/213319, filed on 22 June 2021; 63/260772, filed on 31 August 2021; 63/260776, filed 31 August 2021; 63/260777, filed 31 August 2021; 63/245278, filed 17 September 2021; 63/264059, filed 15 November 2021; 63/264062, filed 15 November 2021; 63/264065, filed 15 November 2021; 15/806014 (US20180124992A1), filed 07 November 2017; and 17/343536, filed on 09 June 2021; and International Application Nos. PCT/IB2021/051076, filed on 10 February 2021; and PCT Application Nos. PCT/IB2021/051077, filed on 10 February 2021; PCT/IB2021/052874, filed on 07 April 2021; PCT/IB2021/052875, filed on 07 April 2021; and PCT/IB2021/052876, filed on 07 April 2021; PCT/IB2021/052872, filed 10 July 2019; PCT/IB2021/054344, filed 20 May 2021; PCT/IB2021/054345, filed 20 May 2021; PCT/IB2021/054346, filed 20 May 2021; PCT/IB2021/054347, filed 20 May 2021; PCT/IB2021/054348, filed 20 May 2021; PCT/IB2021/054349, filed 20 May 2021; PCT/IB2021/054350, filed 20 May 2021; PCT/IB2021/054351, filed 20 May 2021; PCT/IB2021/054352, filed 20 May 2021; PCT/IB2021/054353, filed 20 May 2021; and PCT/IB2021/054354, filed 20 May 2021. The microfluidic manifold chemical analysis substrate 6000 disclosed herein is usable in conjunction with any of the system and devices disclosed in the foregoing patent documents, and others.

The sample preparation sub-system 3002 generally performs the functions of receiving the soil sample cores in a mixer-filter apparatus, volumetric/mass quantification of the soil sample, adding a predetermined quantity or volume of filtered water based on the volume/mass of soil, and mixing the soil and water mixture to produce a soil sample slurry, removing or transferring the slurry from mixer-filter apparatus, and self-cleaning the mixer-filter apparatus for processing the next available soil sample. In some embodiments, the filter may be separate from the mixer.

The chemical analysis sub-system 3003 generally performs the functions of receiving the soil slurry from a mixer-filter apparatus of sub-system 3002, adding extractant, mixing the extractant and slurry in a first chamber to pull out the analytes of interest (e.g. plant available nutrients), centrifuging the extractant-slurry mixture to produce a clear liquid or supernatant, removing or transferring the supernatant to a second chamber, injecting a reagent, holding the supernatant-reagent mixture for a period of hold time to allow complete chemical reaction with reagent, measure the absorbance such as via colorimetric analysis, and assist with cleaning the chemical analysis equipment. In some embodiments, the chemical analysis sub-system 3003 may be embodied in a microfluidic device or apparatus, as further described herein.

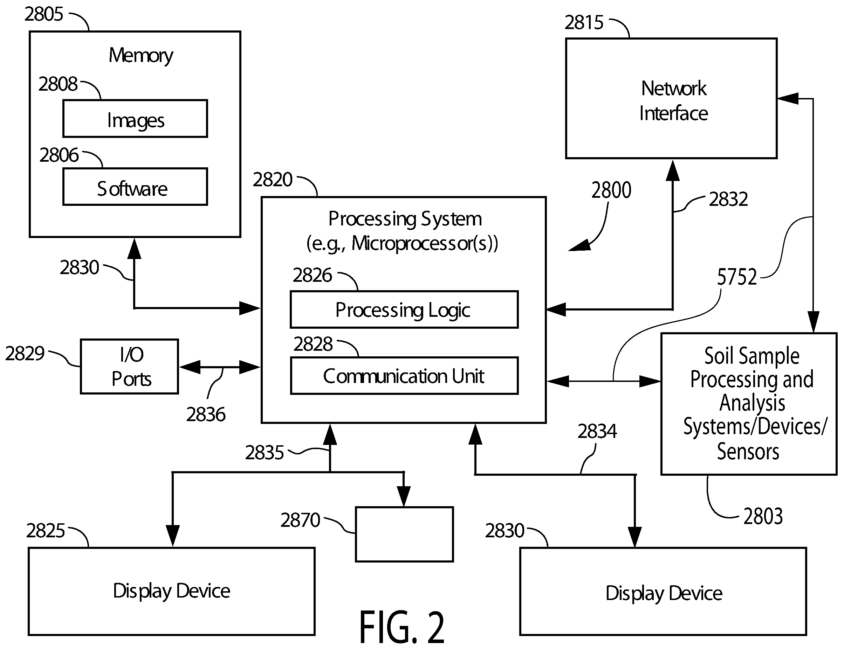

The process described below and in the flow diagrams (see, e.g., FIG. 1) may be automatically controlled and executed by the programmable system controller 2820. The controller may be part of a controller processing system such as that further described herein and shown in FIG. 2, or as disclosed in copending U.S. Patent Application Publication No. 2018/0124992A1. The controller 2820 is operably coupled to the components of the chemical analysis sub-system 3003 disclosed herein (e.g., pumps, valves, centrifuge, compressor (air supply), etc.) for controlling the process sequence and flow of fluids (e.g., water, air, slurry, extractant, reagent, supernatant, etc.) through the system to fully process and analyze the soil or other type agricultural sample. FIG. 2 depicts one embodiment of a programmable system controller 2820 applicable to the present application.

### Supernatant Separators

In some embodiments, the liquid portion may be separated from the soil sample slurry and extractant mixture to produce clear supernatant for chemical analysis using a centrifuge or suitable filter media such an ultrafine microporous filter in lieu of the centrifuge. Suitable centrifuges include centrifuge 3400 and centrifuge tubes described in commonly-owned WO2020/012369. A microporous filter 5757 instead for producing the supernatant include ones such as those described in commonly-owned International Application No. PCT/IB2021/052872 and U.S. Patent Application No. 17/343434 filed June 9, 2021. In some embodiments, a microporous sintered metal filter media of suitable shape and structure may be used for the microporous filter. Preferably, the filter media material and shape selected are suitable for backwashing. The microporous filter media selected is configured to produce a clear supernatant suitable for chemical analysis from the slurry and extractant mixture which is suitable for chemical in the microfluidic manifold chemical analysis substrate 6000 further described herein.

The agricultural sampling system, sub-systems, and related processes/methods disclosed herein may be used for processing and testing soil, vegetation/plants, manure, feed, milk, or other agricultural materials for related parameters of interest. Particularly, embodiments of the chemical analysis portion of the system (chemical analysis sub-system 3003) disclosed herein can be used to test for multitude of chemical-related parameters and analytes (e.g. nutrients/chemicals of interest) in other areas beyond soil and plant/vegetation sampling. Some non-limiting examples (including soil and plants) are as follows.
Soil Analysis: Nitrate, Nitrite, Total Nitrogen, Ammonium, Phosphate, Orthophosphate, Polyphosphate, Total Phosphate, Potassium, Magnesium, Calcium, Sodium, Cation Exchange Capacity, pH, Percent Base Saturation of Cations, Sulfur, Zinc, Manganese, Iron, Copper, Boron, Soluble Salts, Organic Matter, Excess Lime, Active Carbon, Aluminum, Amino Sugar Nitrate, Ammoniacal Nitrogen, Chloride, C:N Ratio, Electrical Conductivity, Molybdenum, Texture (Sand, Silt, Clay), Cyst nematode egg counts, Mineralizable Nitrogen, and Soil pore space.
Plants/Vegetation: Nitrogen, Nitrate, Phosphorus, Potassium, Magnesium, Calcium, Sodium, Percent Base Saturation of Cations, Sulfur, Zinc, Manganese, Iron, Copper, Boron, Ammoniacal Nitrogen, Carbon, Chloride, Cobalt, Molybdenum, Selenium, Total Nitrogen, and Live plant parasitic nematode.
Manure: Moisture/Total Solids, Total Nitrogen, Organic Nitrogen, Phosphate, Potash, Sulfur, Calcium, Magnesium, Sodium, Iron, Manganese, Copper, Zinc, pH, Total Carbon, Soluble Salts, C/N Ratio, Ammoniacal Nitrogen, Nitrate Nitrogen, Chloride, Organic Matter, Ash, Conductance, Kjeldahl Nitrogen, E.coli, Fecal Coliform, Salmonella, Total Kjeldahl Nitrogen, Total Phosphate, Potash, Nitrate Nitrogen, Water Soluble Nitrogen, Water Insoluble Nitrogen, Ammoniacal Nitrogen, Humic Acid, pH, Total Organic Carbon, Bulk Density (packed), Moisture, Sulfur, Calcium, Boron, Cobalt, Copper, Iron, Manganese, Arsenic, Chloride, Lead, Selenium, Cadmium, Chromium, Mercury, Nickel, Sodium, Molybdenum, and Zinc
Feeds: Alanine, Histidine, Proline, Arginine, Isoleucine, Serine, Aspartic Acid, Leucine, Threonine, Cystine, Lysine, Tryptophan, Glutamic Acid, Methionine, Tyrosine, Glycine, Phenylalanine, Valine (Requires Crude Protein), Arsenic, Lead, Cadmium, Antimony, Mercury
Vitamin E (beta-tocopherol), Vitamin E (alpha-tocopherol), Vitamin E (delta-tocopherol), Vitamin E (gamma-tocopherol), Vitamin E (total), Moisture, Crude Protein, Calcium, Phosphorus, ADF, Ash, TDN, Energy (Digestible and Metabolizable), Net Energy (Gain, Lactation, Maintenance), Sulfur, Calcium, Magnesium, Sodium, Manganese, Zinc, Potassium, Phosphorus, Iron, Copper (not applicable to premixes), Saturated Fat, Monounsaturated Fat, Omega 3 Fatty Acids, Polyunsaturated Fat, Trans Fatty Acid, Omega 6 Fatty Acids (Requires Crude or Acid Fat), Glucose, Fructose, Sucrose, Maltose, Lactose, Aflatoxin (B1, B2, G1, G2), DON, Fumonisin, Ochratoxin, T2-Toxin, Zearalenone, Vitamin B2, B3, B5, B6, B7, B9, and B12, Calories, Chloride, Crude fiber, Lignin, Neutral Detergent Fiber, Non Protein Nitrogen, Selenium U.S. Patent, Total Iodine, Total Starch, Vitamin A, Vitamin D3, and Free Fatty Acids.
Forages: Moisture, Crude Protein, Acid Detergent Fiber ADF, NDF, TDN, Net Energy (Gain, Lactation, Maintenance), Relative Feed Value, Nitrate, Sulfur, Copper, Sodium, Magnesium, Potassium, Zinc, Iron, Calcium, Manganese, Sodium, Phosphorus, Chloride, Fiber, Lignin, Molybdenum, Prussic Acid, and Selenium USP.
Milk: Butterfat, True Protein, Somatic Cell Count, Lactose, Other Solids, Total Solids, Added Water, Milk Urea Nitrogen, Acidity, pH, Antibiotic tests, and Micro-organisms.

### Control System

FIG. 2 is a schematic system diagram showing the control or processing system 2800 including programmable processor-based central processing unit (CPU) or system controller 2820 as referenced to herein. System controller 2820 may include one or more processors, non-transitory tangible computer readable medium, programmable input/output peripherals, and all other necessary electronic appurtenances normally associated with a fully functional processor-based controller. Control system 2800, including controller 2820, is operably and communicably linked to the different soil sample processing and analysis systems and devices described elsewhere herein via suitable communication links to control operation of those systems and device in a fully integrated and sequenced manner.

Referring to FIG. 2, the control system 2800 including programmable controller 2820 may be mounted on a stationary support in any location or conversely on a translatable self-propelled or pulled machine (e.g., vehicle, tractor, combine harvester, etc.) which may include an agricultural implement (e.g., planter, cultivator, plough, sprayer, spreader, irrigation implement, etc.) in accordance with one embodiment. In one example, the machine performs operations of a tractor or vehicle that is coupled to an implement for agricultural operations. In other embodiments, the controller may be part of a stationary station or facility.

Control system 2800, whether onboard or off-board a translatable machine, generally includes the controller 2820, non-transitory tangible computer or machine accessible and readable medium such as memory 2805, and a network interface 2815. Computer or machine accessible and readable medium may include any suitable volatile memory and non-volatile memory or devices operably and communicably coupled to the processor(s). Any suitable combination and types of volatile or non-volatile memory may be used including as examples, without limitation, random access memory (RAM) and various types thereof, read-only memory (ROM) and various types thereof, hard disks, solid-state drives, flash memory, or other memory and devices which may be written to and/or read by the processor operably connected to the medium. Both the volatile memory and the non-volatile memory may be used for storing the program instructions or software. In one embodiment, the computer or machine accessible and readable non-transitory medium (e.g., memory 2805) contains executable computer program instructions which when executed by the system controller 2820 cause the system to perform operations or methods of the present disclosure including measuring properties and testing of soil and vegetative samples. While the machine accessible and readable non-transitory medium (e.g., memory 2805) is shown in an exemplary embodiment to be a single medium, the term should be taken to include a single medium or multiple media (e.g., a centralized or distributed database, and/or associated caches and servers) that store the one or more sets of control logic or instructions. The term "machine accessible and readable non-transitory medium" shall also be taken to include any medium that is capable of storing, encoding or carrying a set of instructions for execution by the machine and that cause the machine to perform any one or more of the methodologies of the present disclosure. The term "machine accessible and readable non-transitory medium" shall accordingly also be taken to include, but not be limited to, solid-state memories, optical and magnetic media, and carrier wave signals.

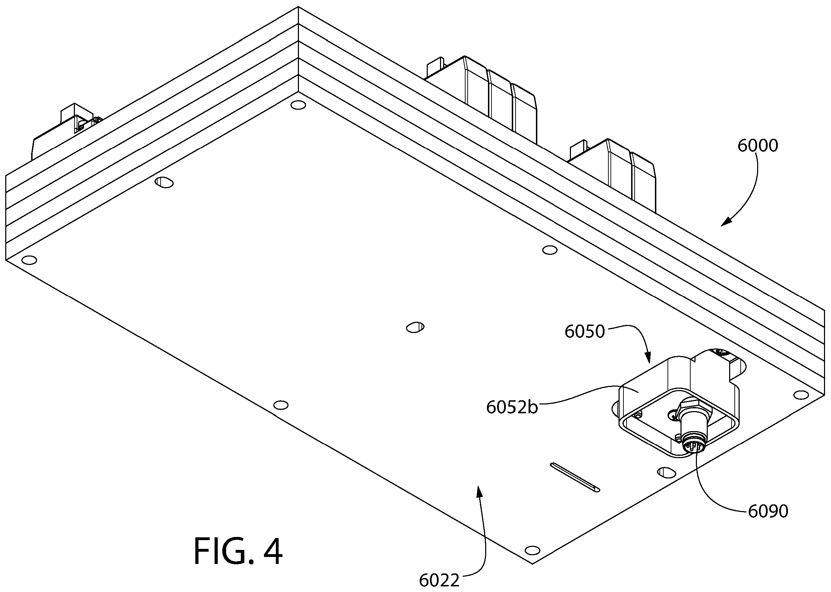

Network interface 2815 communicates with the agricultural (e.g. soil or other) sample processing and analysis systems (and their associated devices) described elsewhere (collectively designated 2803 in FIG. 4), and other systems or devices which may include without limitation implement 2840 having its own controllers and devices. The sample and analysis systems 2803 therefore specifically include devices such as but are not limited to the chemical analysis substrate 6000 and process performed by this device which may be sequenced and controlled by controller 2820.

The programmable controller 2820 may include one or more microprocessors, processors, a system on a chip (integrated circuit), one or more microcontrollers, or combinations thereof. The processing system includes processing logic 2826 for executing software instructions of one or more programs and a communication module or unit 2828 (e.g., transmitter, transceiver) for transmitting and receiving communications from network interface 2815 and/or agricultural sample processing and analysis system 2803 which includes sample preparation sub-system 3002 and the components described herein further including the closed slurry recirculation flow loop 8002 components. The communication unit 2828 may be integrated with the control system 2800 (e.g. controller 2820) or separate from the programmable processing system.

Programmable processing logic 2826 of the control system 2800 which directs the operation of system controller 2820 including one or more processors may process the communications received from the communication unit 2828 or network interface 2815 including agricultural data (e.g., test data, testing results, GPS data, liquid application data, flow rates, etc.), and soil sample processing and analysis systems 2803 generated data. The memory 2805 of control system 2800 is configured for preprogrammed variable or setpoint/baseline values, storing collected data, and computer instructions or programs for execution (e.g. software 2806) used to control operation of the controller 2820. The memory 2805 can store, for example, software components such as testing software for analysis of soil and vegetation samples for performing operations of the present disclosure, or any other software application or module, images2808 (e.g., captured images of crops), alerts, maps, etc. The system 2800 can also include an audio input/output subsystem (not shown) which may include a microphone and a speaker for, for example, receiving and sending voice commands or for user authentication or authorization (e.g., biometrics).

The system controller 2820 communicates bi-directionally with memory 2805 via communication link 2830, network interface 2815 via communication link 2832, display device 2830 and optionally a second display device 2825 via communication links 2834, 2835, and I/O ports 2829 via communication links 2836. System controller 2820 may further communicate with the soil sample processing and analysis systems 2803 via wired/wireless communication links 5752 either via the network interface 2815 and/or directly as shown.

Display devices 2825 and 2830 can provide visual user interfaces for a user or operator. The display devices may include display controllers. In one embodiment, the display device 2825 is a portable tablet device or computing device with a touchscreen that displays data (e.g., test results of soil, test results of vegetation, liquid application data, captured images, localized view map layer, high definition field maps of as-applied liquid application data, as-planted or as-harvested data or other agricultural variables or parameters, yield maps, alerts, etc.) and data generated by an agricultural data analysis software application and receives input from the user or operator for an exploded view of a region of a field, monitoring and controlling field operations. The operations may include configuration of the machine or implement, reporting of data, control of the machine or implement including sensors and controllers, and storage of the data generated. The display device 2830 may be a display (e.g., display provided by an original equipment manufacturer (OEM)) that displays images and data for a localized view map layer, as-applied liquid application data, as-planted or as-harvested data, yield data, controlling a machine (e.g., planter, tractor, combine, sprayer, etc.), steering the machine, and monitoring the machine or an implement (e.g., planter, combine, sprayer, etc.) that is connected to the machine with sensors and controllers located on the machine or implement.

### Microfluidic Sample Analysis System with Temperature Compensation

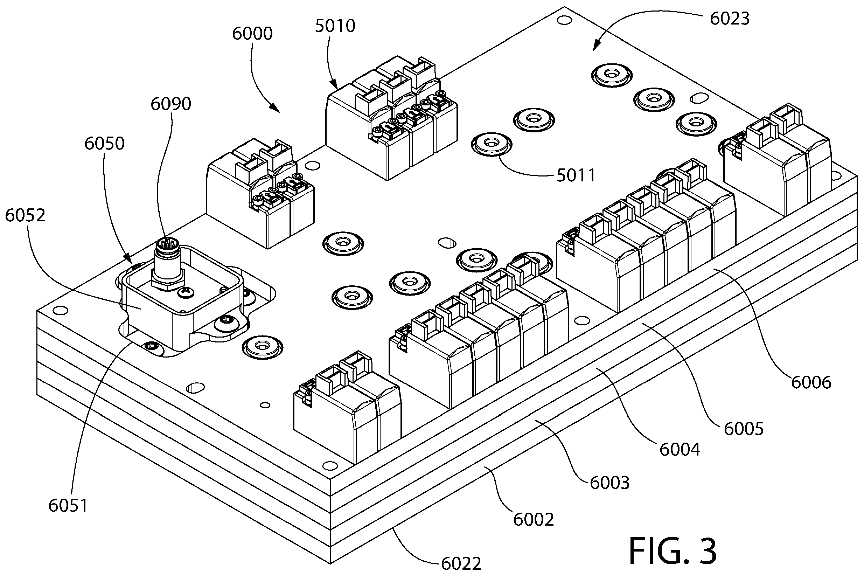

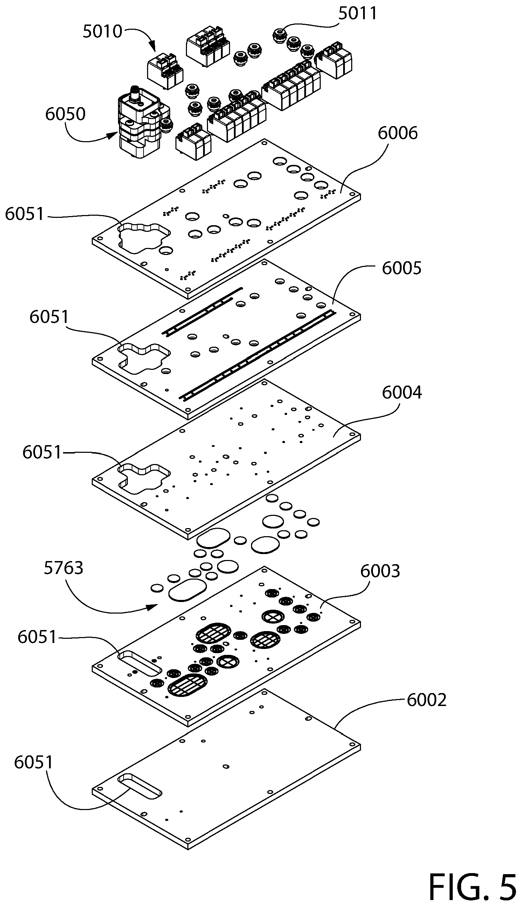

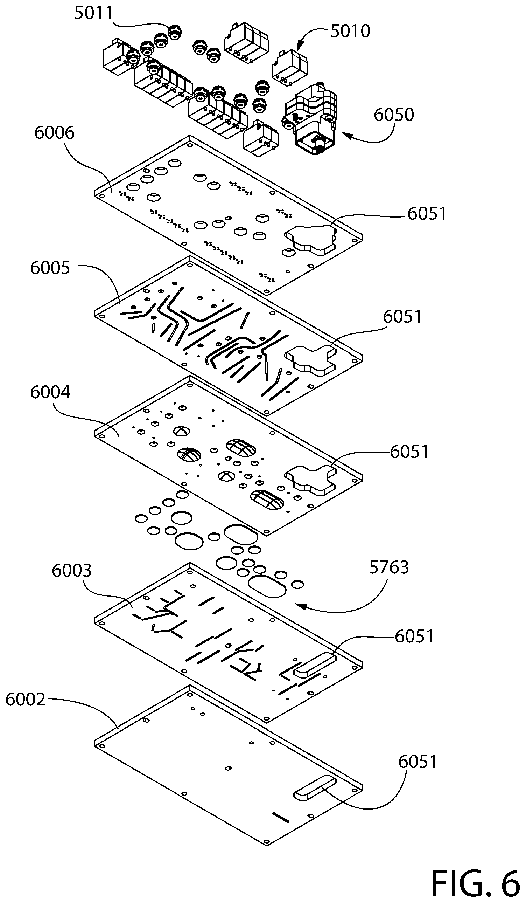

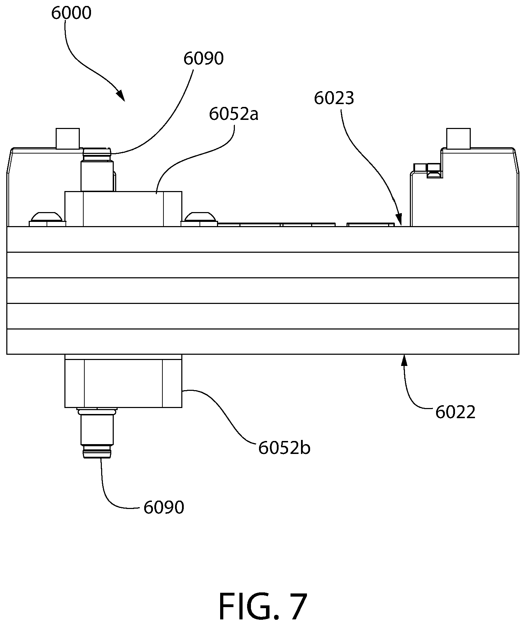

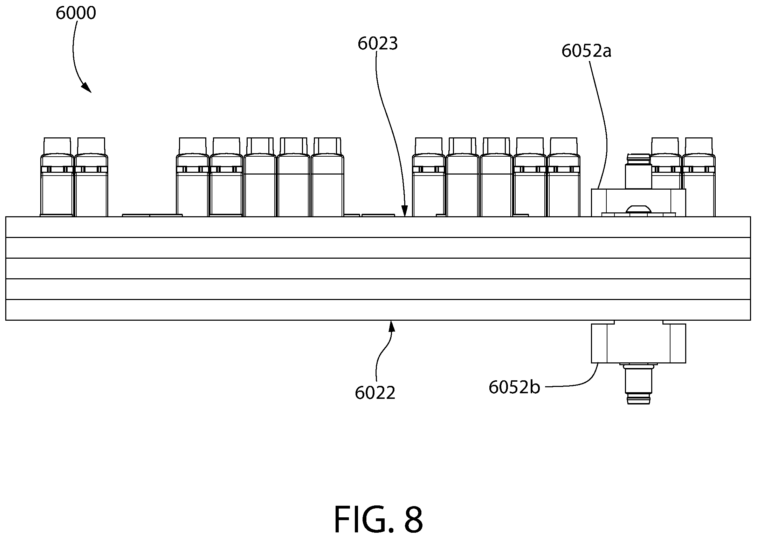

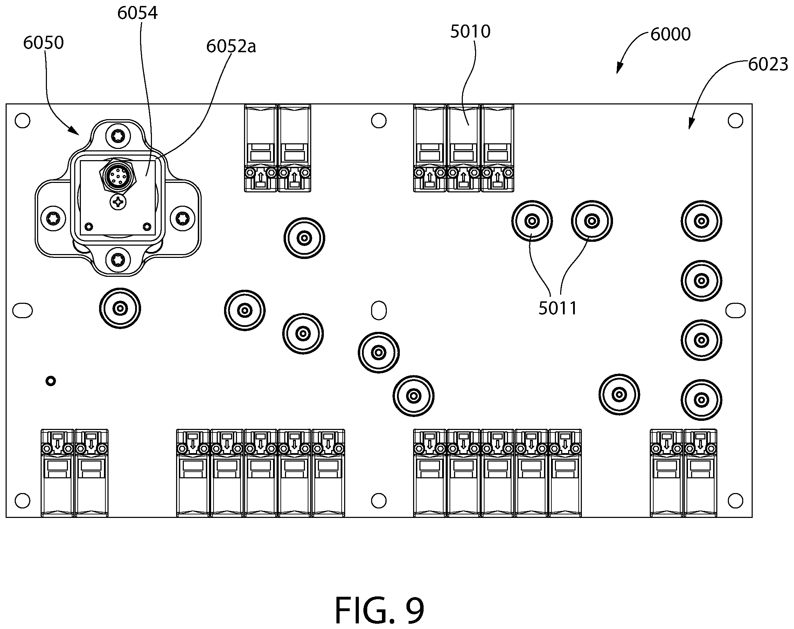

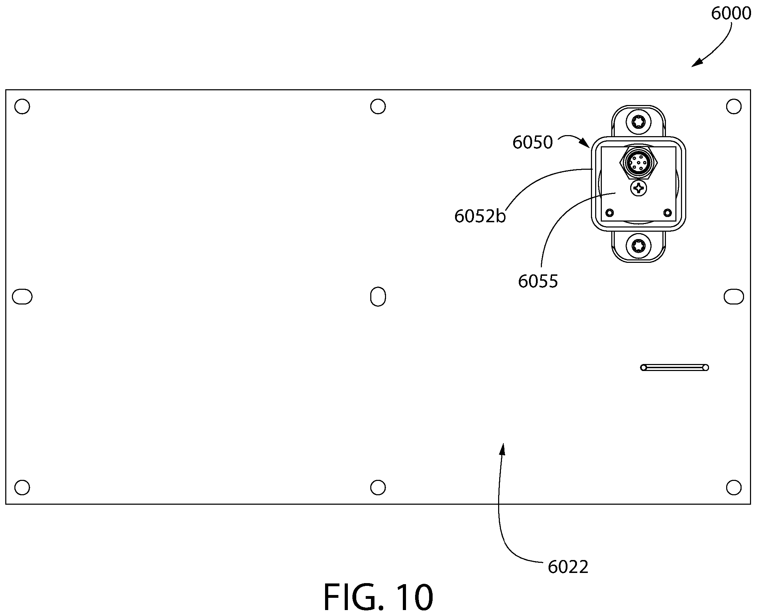

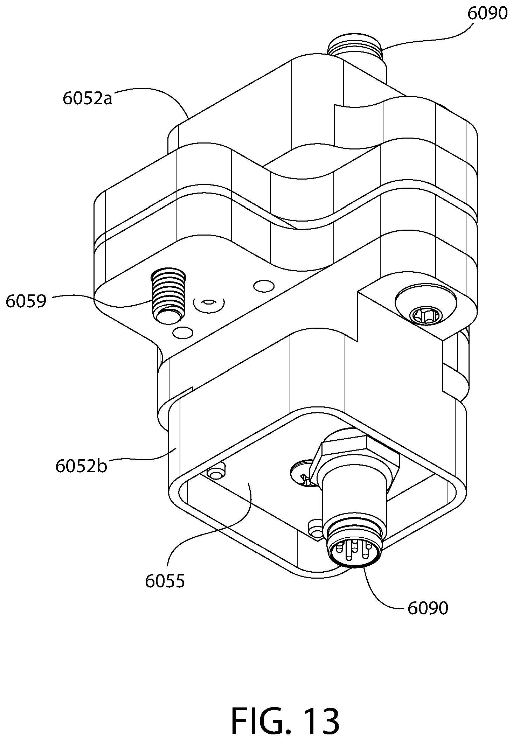

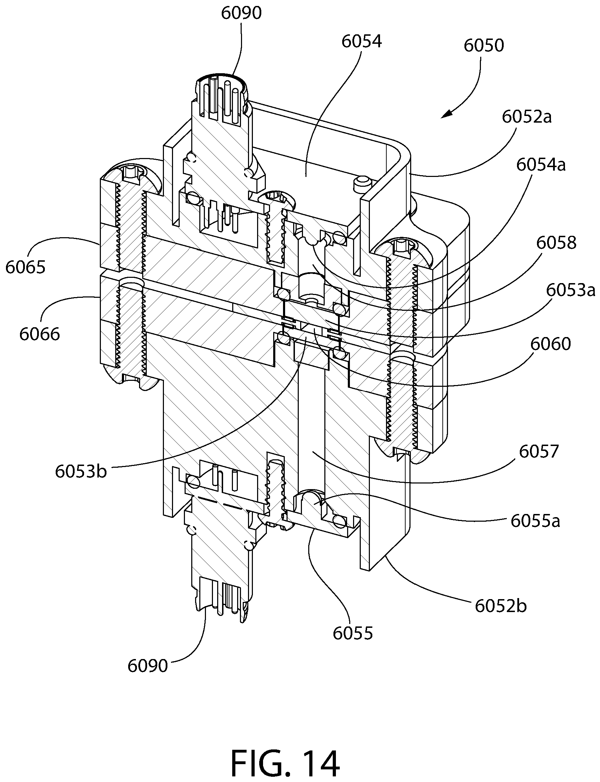

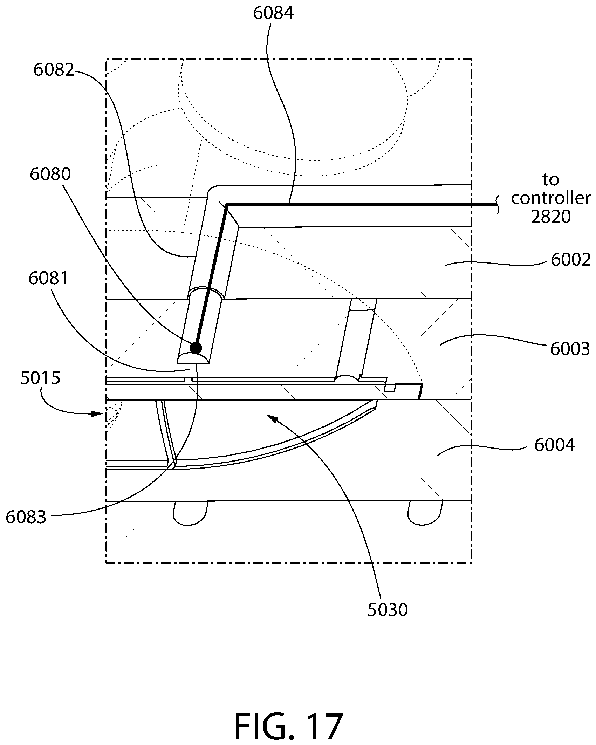

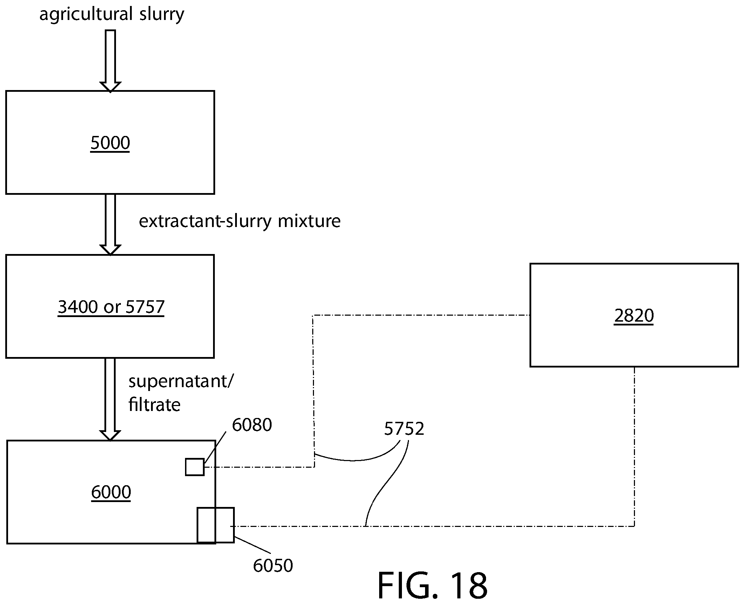

FIGS. 3-20 show various aspects of an embodiment of a microfluidic manifold comprising a polygonal shaped microfluidic manifold chemical analysis substrate 6000 configured for chemically analyzing the agricultural slurry sample separately processed to produce a supernatant by any suitable means, including for example without limitation by the slurry processing substrate 5000 disclosed in commonly-owned U.S Provisional Application No. 63/213,319 filed June 22, 2021 and U.S. Patent Application No. 17/343,536 filed June 9, 2021; or the centrifuge 3400 described in commonly-owned WO2020/012369. The supernatant separation apparatuses are represented schematically in the block diagram of FIG. 18.

Analysis substrate 6000 is multi-layer structure which comprises a plurality of layers 6001 which may be permanently bonded together via adhesives, thermal/heat bonding, or other fabrication techniques previously described herein.

In the present embodiment, the functions of extracting the analyte from the agricultural slurry and then subsequently analyzing the centrifugated or filtered slurry (e.g., supernatant) for the concentration of the analyte of interest or other chemical property of the slurry (e.g., pH or BpH) are separated into two discrete and dedicated microfluidic manifold devices. FIG. 18 is a system block diagram of the overall agricultural sample processing and analysis system. For example, the extractant and slurry mixing function to prepare the sample fluid for chemical analysis by producing the supernatant may be performed by microfluidic manifold slurry processing substrate 5000 alone. The reagent and supernatant mixing function as well as the analysis and measurement of analyte in the sample fluid may be performed by the chemical analysis substrate 6000. Substrate 6000 includes the reagent/supernatant mixture measurement device such as the flow/slurry analysis cell 4027 which may be formed by optical absorbance measurement device 6050 in one embodiment. Device 6050 is configured to measure and quantify the concentration (e.g. ppm - parts per million) of analyte in the sample fluid. The analysis substrate 6000 may receive the supernatant from either centrifuge 3400 or ultrafine microporous filter 5757 fluidly coupled between the substrates 5000 and 6000. In the latter case, the supernatant may be considered a filtrate from microporous filter 5757.

The term "sample fluid" as used herein shall be construed to broadly connote a fluid derived from the agricultural sample such as the reagent-supernatant mixture, extractant-slurry mixture, raw slurry formed of the agricultural material and a carrier such as water, etc.

The chemical analysis substrate 6000 may generally include the same flow control devices and flow conduits such as the internal pneumatically-actuated microfluidic devices previously described for slurry processing substrate 5000 in commonly-owned U.S Provisional Application No. 63/213,319 filed June 22, 2021.

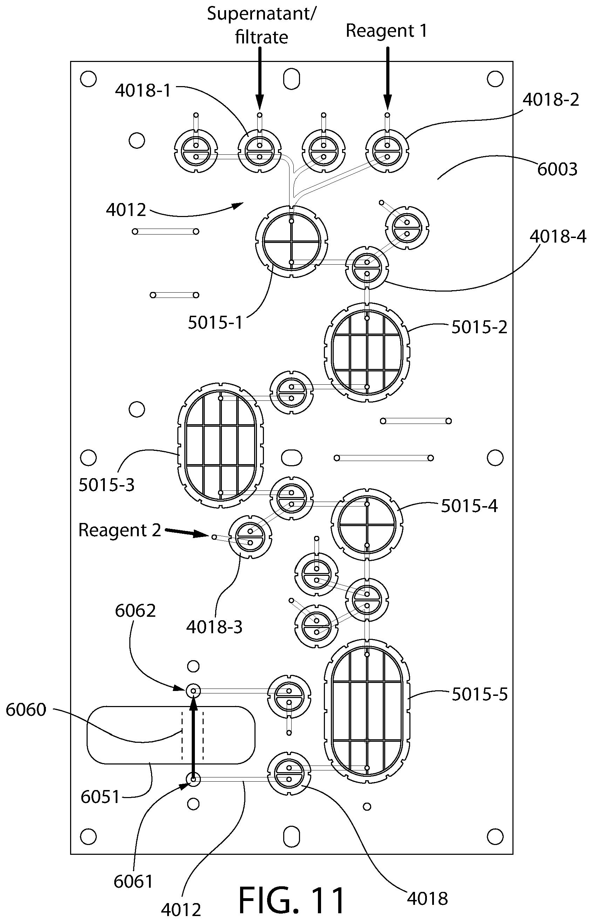

This includes plural diaphragm-operated micropumps 5015 and microvalves 4018, a chemical analysis cell or device 4027, and a network of branched microchannels 4012 fluidly coupling the devices together. FIG. 11 shows one non-limiting embodiment and arrangement of fluid interconnections between the microfluidic devices.

Similarly to slurry processing substrate 5000, the chemical analysis substrate 6000 in one non-limiting embodiment may have a five-layer sandwiched construction recognizing that more of less layers may be provided in other embodiments as needed depending on the type of agricultural slurry processing intended to be performed. In order from the planar outer first major surface or side 6022 to opposite planar outer second major surface or side 6023, the adjacent layers of the packaged analysis substrate 6000 include first outer layer 6002, liquid layer 6003 thereon, air layer 6004 thereon, fluid distribution layer 6005 (e.g., air and liquid including but not limited to supernatant, reagent, mixtures thereof, pressurized cleaning/flushing water, etc.) thereon, and second outer layer 6006 thereon. Outer layer 6002 defines first major surface or side 6022 while opposite outer layer 6006 defines second major surface or side 6023. The remaining layers of substrate 6000 are inner layers. The substrate further includes top side 6020, opposite bottom side 6021, and pair of opposed lateral sides 6024. Major surfaces or sides 6022, 6023 have a greater surface area than other sides of the microfluidic manifold chemical analysis substrate 6000. It bears noting that the foregoing layer and side designation use the same reference numbers as slurry processing substrate 5000 except 6000 series numbers are assigned in lieu of the original 5000 series numbering. This shows the correlation between the parts of the two substrates but slightly different configuration and layout of each layer and its constituent components due to their different functions.

Outer layer 6006 includes a plurality of quick-connect liquid fittings 5011 and quick connect air valves 5010 similarly to slurry processing substrate 5000. Fluid distribution layer 6005 is adjacent outer layer 6006 and includes a plurality of both fluidly separate and/or interconnected microchannels 4012 for transferring the air and liquids from their applicable sources via fittings 5010, 5011 to and in turn the microfluidic devices (e.g., microvalves 4018 and micropumps 5015) in the microfluidic manifold chemical analysis substrate 6000. Each micropump 5015 and microvalve 4018 in certain non-limiting embodiments comprises an individual thin and resiliently deformable elastomeric diaphragm 5763 having an elastic memory. The underside of liquid layer 6003 comprises a plurality of microchannels 4012 which fluidly couple the microvalves 4018 and micropumps 5015 together. FIG. 11 shows the fluid interconnections between these microfluidic devices/components formed by the microchannels 4012.

In one non-limiting embodiment, the devices and microchannels of the chemical analysis substrate 6000 may be configured for mixing one or more indicators or reagents (collectively referred to as reagents herein) with the supernatant (filtrated) to create a chemical reaction which changes the color or turbidity of the reagent-supernatant mixture. The reagent operates to change the optical absorbance of light at one or more specified wavelengths.

The absorbance measurement device 6050 configured to measure and quantify the concentration of the analyte (e.g., soil nutrients or other) or other chemical properties such as pH and/or BpH in the agricultural sample fluid.

Microfluidic manifold chemical analysis substrate 6000 may have a rectangular cuboid configuration in one embodiment as shown; however, other polygonal shapes may be used. Chemical analysis substrate 6000 may be used in a vertical orientation in one non-limiting preferred embodiment shown in FIG. 11 to obtain the benefit gravity assisted agricultural sample fluid flow through the substrate from top to bottom. Other vertical orientations of the rectangular cuboid substrate 6000 (e.g., long sides extending horizontal instead of vertically seen in FIG. 51), horizontal orientations, or angled orientations with respect to vertical and horizontal may be used in other implementations.

The materials used to construct the individual main layers of chemical analysis substrate 6000 may include a combination of rigid thermoplastics with flexible elastomeric materials used for the deformable diaphragms associated with each of the micropumps 5015 and microvalves 4018. Transparent polymeric materials may be used in one embodiment to permit visual observation of the fluids being processed in the chemical analysis substrate 6000. The rigid plastics may be used to form the overall rigid substrate or body of chemical analysis substrate 6000 which defines its exposed exterior surfaces and includes an interior patterned to create a plurality of internal microchannels 4012 and chambers for creating the active microfluidic flow control devices (e.g. diaphragm-operated pumps, valves, mixing chambers, etc.). Examples of thermoplastics (polymers) which may be used include for example without limitation PMMA (polymethyl methacrylate commonly known as acrylic), PC (polycarbonate), PS (polystyrene), and others.

Examples of suitable elastomeric materials which may be used for the diaphragms of the microvalves and micropumps include for example without limitation silicone, PDMS (polydimethylsiloxane), fluorosilicone, neoprene, and others. The pressurized air used to hold the microfluidic valves/pumps closed will permeate through elastomeric diaphragms over time, causing bubbles to develop in the liquid side of the device. These bubbles negatively affect the ability to volumize liquids properly, as the air bubbles displace the otherwise precise fluid volumes that are being manipulated. Fluorosilicone is one preferred non-limiting material due its low gas permeability property which aids in decreasing gas diffusion through the diaphragm over time to combat the foregoing problem.

Referring to FIG. 11, the liquid layer 6003 is shown indicating the flow path of the agricultural sample fluid (e.g., supernatant/filtrate, reagent(s), and reagent-supernatant/filtrate mixture or solution. Bolded flow arrows show the different fluid/reagent inlets and flowpath of the sample liquid through the absorbance measurement device (discussed in further detail below). In this present example for convenience of description, it will be assumed that a microporous filter 5757 or centrifuge 3400 is used to produce a clear filtrate or supernatant suitable for chemical analysis by chemical analysis substrate 6000. In either case, a clear liquid is produced containing the analyte so that the terms supernatant and filtrate may be used interchangeably herein to refer to the same clear liquid for chemical analysis and analyte quantification.

With continuing reference to FIG. 11, the micropumps 5015, microvalves 4018, and microchannels 4012 which fluidly coupling these microfluidic devices together are shown in one non-limiting example of numerous possible arrangements. The agricultural sample fluid derived from the agricultural sample progressively flows downwards from top to bottom of the substrate 6000 to take advantage of gravity assisted flow working in unison with the pumped flow driven by the micropumps. The supernatant/filtrate enters the absorbance analysis substrate 6000 in liquid layer 6003 at top via supernatant/filtrate inlet microvalve 4018-1 from the microporous filter 5757. The supernatant/filtrate flows into a first micropump 5015-1 where it is mixed with a first reagent (Reagent 1 in figure) flowing from reagent microvalve 4018-2. The supernatant/filtrate and first reagent mixture is pumped by first micropump 5015-1 to a second micropump 5015-2 via one of several inter-pump microvalves 4018-4 as shown which controls flow (i.e. on/off) between the micropumps (only one inter-pump microvalve being labeled in FIG. 11 for brevity). The supernatant/filtrate and first reagent mixture is pumped by second micropump 5015-2 to a third micropump 5015-3, from which is pumped to a fourth micropump 5015-4. As the supernatant/filtrate and first reagent mixture flows through another inter-pump microvalve 4018-4 between micropumps 5015-3 and 5015-4, it is mixed with a second reagent (Reagent 2 in figure) from reagent microvalve 4018-3 as shown. Micropump 5015-4 then pumps the mixture of the supernatant/filtrate and first-second reagents to a fifth micropump 5015-5. Micropump 5015-5 is the final or last micropump located immediately upstream and proximate to the absorbance measurement device 6050 and measurement passageway 6060 (further described below). Another inter-pump microvalve 4018-4 separates and controls flow between the first micropump 5015-5 and measurement device 6050 as shown. Micropump 5015-5 then pumps the supernatant/filtrate and first-second reagent mixture to the fluid inlet 6061 of the measurement device, the mixture flows through the measurement passageway 6060 for measurement/quantification of the analyte of interest in the mixture, and exits the device via the fluid outlet 6062. In a preferred embodiment, it is highly desirable to have the flow path of the liquid mixture through the measurement passageway 6060 (flow cell) to be in a vertically upwards direction so that both flow and gravity work together to motivate any entrained air or gas bubbles to rise up and out of the absorbance measurement light path (see, e.g. FIG. 11 showing vertical orientation of passageway 6060 represented by dashed lines). The presence of entrained air or gas bubbles in the liquid under optical absorbance analysis in measurement device 6050 can create errors in measuring the concentration/level of analyte. By using the vertically oriented flowpath and upwards flow of the liquid mixture through the measurement passageway 6060, any entrained air/gas bubbles in the measurement passageway rise and are driven out of the line of sight of the optical absorbance measurement device 6050 to present measurement errors.

In the foregoing example, two reagents are used an introduced into the flow of supernatant/filtrate at two different and physically spaced apart locations in the flow path. This allows time for the first reagent (Reagent 1) to mix with the supernatant/filtrate via pumping through three micropumps 5015-1, 5015-2, and 5015-3 prior to adding the second reagent (Reagent 2) in order for the first chemical reaction to be completed and accomplish its intended purpose before introduction of the second reagent and completion of the second reaction followed by subsequent analysis of the mixture in the absorbance measurement device 6050.

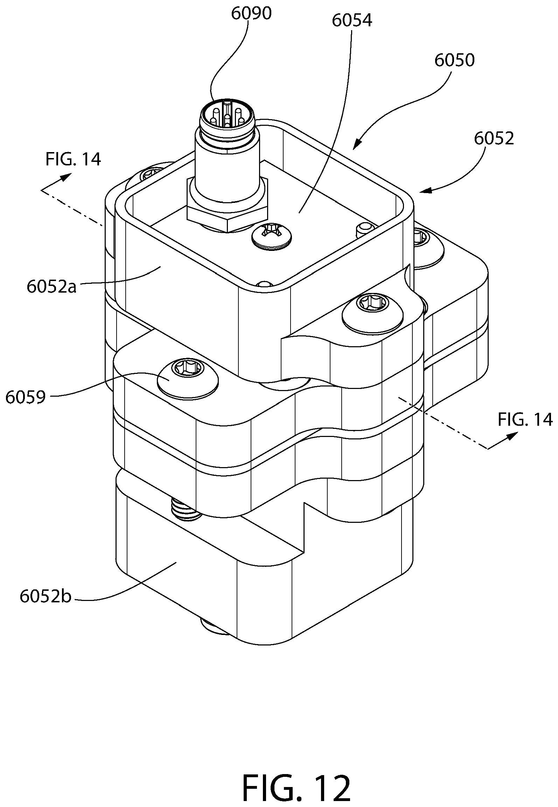

The optical absorbance measurement device 6050 will now be further described with reference to the figures and particularly FIGS. 12-16. In one embodiment, the absorbance measurement device may be onboard and incorporated directly into the chemical analysis substrate 6000 via mounting aperture 6051. Aperture 6051 is a through opening in one embodiment which may extend completely through the multiple layers of the substrate between major sides 6022 and 6023 and is outwardly open from each end to allow portions of the measurement device housing 6052 to protrude outwards from the substrate. Aperture 6051 may have any suitable transverse cross-sectional shape based on the shape of the housing of the measurement device.

Measurement device 6050 generally includes housing 6052 comprised of detector sub-housing 6052a and transmitter sub-housing 6052b, upper and lower support plates 6065, 6066, upper window 6053a, and lower window 6053b. Detector sub-housing 6052a supports light detector printed circuit board (PCB) 6054 removably attached thereto which comprises light detector 6054a. Transmitter sub-housing 6052b supports transmitter PCB 6055 removably attached thereto which comprises light transmitter detector 6055a in axial alignment with the light detector. Transmitter PCB 6055 may be located adjacent to the first major surface or side 6022 and detector PCB 5054 may be located adjacent to second major surface or side 6023 on an opposite side of the analysis substrate 6000. Light transmitter 6055a is mounted in light passage 6057 of the transmitter sub-housing. Light detector 6064a is mounted in light passage 6058 of the detector sub-housing. Light passages 6057, 6058 communicate directly with the upper and lower windows 6053a, 6053b which may be formed of a transparent material such as glass or polymer operable to transmit light therethrough.

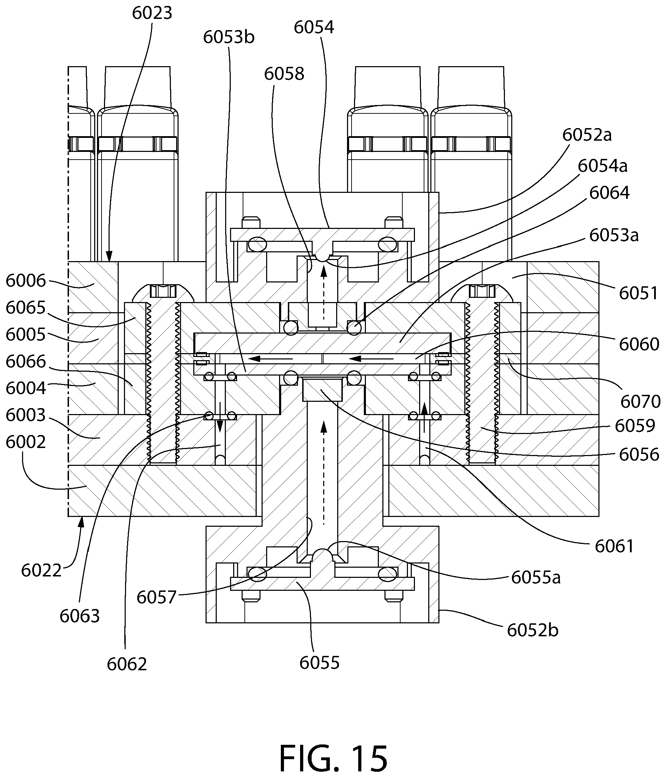

Elongated measurement passageway 6060 defines a flow cell and is formed between the pair of transparent windows 6053a, 6053b for flowing the agricultural sample fluid (e.g., reagent-supernatant mixture) therethrough for detecting and measuring the analyte absorbance equated to the concentration (e.g., ppm) of analyte present in the fluid. The passageway may be formed in one embodiment by spacer 6070 disposed between the upper and lower support plates 6065, 6066. A fluid inlet 6061 conveys the sample fluid from one of the microchannels 4012 into the measurement passageway 6060. Fluid outlet 6062 receives and conveys the sample fluid from the measurement passageway 6060 back into one of the microchannels 4012. The fluid inlet and outlet may be at opposite ends of the measurement passageway to extend the flow time of the fluid therethrough. The light transmitter 6055a is arranged to transmit the light through the windows and measurement passageway to the detector 6054a (best shown in FIG. 15 - note dashed light arrows and solid fluid flow arrows).

Detector sub-housing 6052a and transmitter sub-housing 6052b may be fluidly sealed to the upper and lower windows 6053a, 6053b by seal rings 6064 which may be an elastomeric O-ring in some embodiments. Seal rings 6063 (e.g., O-rings) seal the fluid inlet and outlets 6061, 6062 to upper and lower support plates 6065, 6066.

In one embodiment, the optical absorbance measurement device 6050 may be detachably mounted to analysis substrate 6000 by threaded fasteners 6059. Fasteners 6059 may pass through the upper and lower support plates 6065, 6066 and threadably engage one of the layers (e.g., liquid layer 6003 in the illustrated embodiment). The fasteners further serve to compress the upper and lower support plates and upper and lower together forming the sandwiched structure shown in FIG. 15.

The transmitter PCB 6055 is configured with the circuitry and electronic devices necessary to generate and transmit the light beam through the sample fluid. Similarly, detector PCB 6056 is configured with the circuitry and electronic devices necessary to receive and process the detected or incident light beam on detector 6054a after passing through the sample fluid in measurement passageway 6060 to generate an absorbance value associated with the analyte being measured (e.g., soil nutrient or other). Absorbance is a dimensionless value and often referred to as simply an absorbance value or unit. Each PCB 6055, 6056 has an associated electronics cable connector 6090 which provides operable coupling of electric power to the boards for the devices therein including the transmitter and detector, and two-way communication links between the system controller 2820 and board electronics (see, e.g. FIG. 18).

Optical absorbance measurement device 6050 operates in a known manner similar to commercially-available absorbance measurement or spectrophotometric devices. Measurement device 6050 is configured and operable to measure the absorbance of light passing through the agricultural sample fluid which can be equated to concentration of the analyte of interest (e.g., ppm).

The chemical reaction that takes place to create absorbance in the agricultural sample fluid (e.g., reagent-supernatant mixture or solution) can vary with temperature. For example, these chemical reactions occur faster at higher fluid temperatures and slower at lower fluid temperatures. Accordingly, an absorbance measurement taken by optical absorbance measurement device 6050 at one temperature when equated to a specific concentration of analyte in the sample fluid might not accurately reflect the actual concentration.

According to one aspect of the present disclosure, an automated analysis system comprising programmable controller 2820 operating in conjunction with optical absorbance measurement device 6050 and temperature sensor 6080 is provided which is configured to generate a temperature-compensated concentration of analyte in the agricultural sample fluid. Advantageously, this results in more accurate determination of the actual concentration of the analyte (e.g., soil nutrient or other chemical) present in the sample fluid than an absorbance measurement obtained without regard for the temperature of the fluid.

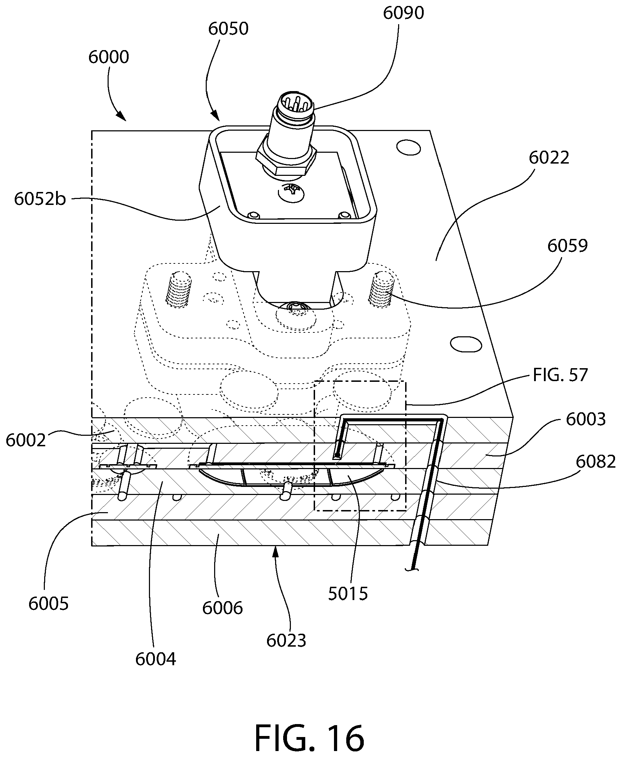

Temperature sensor 6080 may be any suitable commercially-available temperature sensor such as a thermistor or thermocouple. Temperature sensor 6080 may be embedded internally in the substrate proximate to but not necessarily contacting the sample liquid. Accordingly, sensor 6080 may be preferably located upstream of absorbance measurement device 6050 and disposed proximate to in any of the microchannels 4012, micropumps 5015, or microvalves 4018. To accurately measure the temperature of the agricultural sample fluid (e.g., reagent-supernatant solution), sensor 6080 is preferably upstream of absorbance measurement device in close proximity thereto. In one non-limiting embodiment, temperature sensor 4080 may be disposed proximate to the final micropump 5015 upstream of measurement device 6050 to measure the temperature of the agricultural sample fluid in the pump. Sensor 4080 may be separated from the pump chamber 5030 by a partition wall 6081 formed by a portion of the substrate (e.g., liquid layer 6003 in one embodiment as best shown in FIGS. 16-17). In other possible embodiments, the temperature sensor may be located proximate to one of the microchannels 4012 between the final micropump 5015 and measurement device 6050, or the microvalve 4018 between the final micropump and measurement device. In some embodiments, the temperature sensor 6050 may be in physical and direct contact with the agricultural sample fluid rather than separated therefrom by a partition wall.

Referring to FIGS. 16-17, temperature sensor 6080 is mounted in a measurement bore 6082 formed and extending through the layers of the analysis substrate 6000 to the final micropump 5015, a microchannel 4012, or microvalve 4018. In one embodiment, bore 6082 may have a cylindrical configuration with round cross-sectional shape and completely penetrates one of the outer layers 6006 or 6002. In one embodiment, bore 6082 may penetrate outer layer 6006 as shown. Temperature sensor 6080 is disposed at the terminal closed end 6083 of measurement bore 6082 adjacent to partition wall 6081.

Measurement bore 6082 permits the wire leads 6084 of temperature sensor 6080 to be routed from the sensor through the substrate layers to system controller 2820 as shown in the system block diagram of FIG. 18. Sensor 6080 is configured to measure a real-time temperature of the agricultural sample fluid and transmit the measure real-time temperature to the controller. As shown, the optical absorbance measurement device 6050 is also operably coupled to system controller 2820 for transmitting the measured absorbance of light thereto which is indicative of the concentration of analyte present in the sample fluid. The controller uses the real-time temperature and absorbance value to execute a software routine which generates a temperature-compensated concentration of analyte.

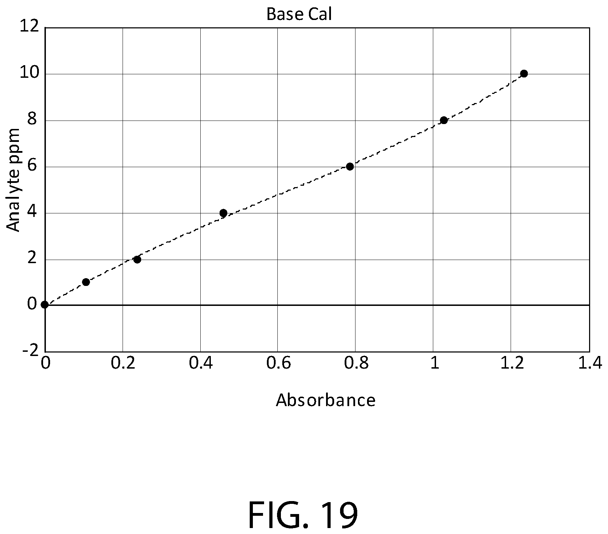

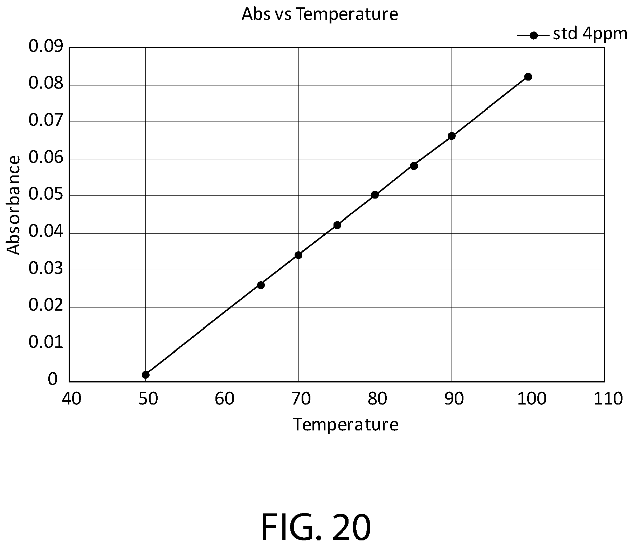

To determine the temperature-compensated concentration of analyte, the controller may use and is preprogrammed with a set of curves similar to those shown in the graphs of FIGS. 19 and 20. FIG. 19 is a graph of a base calibration curve showing absorbance units on one scale versus concentration of a selected analyte (ppm) on the other scale (e.g., phosphorous, nitrogen, potassium, etc.). This curve is used by controller 2820 to correlate a measured absorbance obtained by optical absorbance measurement device 6050 to a concentration of the selected analyte in the agricultural sample fluid. The graph is constructed by conducting multiple test runs to measure the absorbance of a plurality of different calibration standard fluids (or simply "standards") each having a different and unique known concentration (ppm) of the selected test analyte of interest.

FIG. 20 is a graph showing absorbance units on one scale versus temperature on the other scale. Shown is one example of a temperature compensation curve developed empirically by testing a first calibration standard fluid having a known concentration (ppm) of the selected analyte (e.g., 4 ppm of phosphorous in the example graph) over a range of different temperatures which might be encountered when testing the agricultural sample fluid in measurement device 6050. Accordingly, the curve indicates the temperature compensation relationship of the selected analyte for the first calibration standard fluid of known concentration. The temperature compensation curve shows the shift in absorbance values/units as the sample fluid temperature changes. The temperature compensation curve may be developed by running the first calibration standard fluid through the analysis substrate 6000 and obtaining measurements from the measurement device 6050 and temperature sensor 6080.

Although only a single curve is shown in FIG. 20 for simplicity, a plurality of different temperature compensation curves would be generated in a similar manner for each of a multitude of different calibration standard fluids each having a unique and different known concentration of the selected analyte which were originally used to construct the base calibration curve of FIG. 19. The base calibration curve may be automatically adjusted by controller 2820 in real-time based on the real-time temperature measurements from temperature sensor 6080 and the plural temperature compensation curves indicating the variance of absorbance values/units with temperature for the different calibration standards. Thus, the base calibration curve is adjusted up or down in real-time by controller 2820 to compensate for the real-time temperature of the agricultural sample fluid measured by temperature sensor 6080 which is communicated to the controller.

In operation when testing an agricultural sample fluid for the concentration of an analyte in microfluidic manifold analysis substrate 6000, optical absorbance measurement device 6050 measures the absorbance of the sample fluid. The absorbance value is transmitted to system controller 2820 along with a corresponding real-time temperature measure of the fluid captured by temperature sensor 6080. The controller adjusts the base calibration curve as needed based on the measured real-time temperature using graph of FIG. 20. Using the temperature-compensated base calibration curve, the controller then used the graph of FIG. 19 to correlate the measured absorbance of the selected analyte to a corresponding concentration of the analyte (e.g., ppm). Accordingly, the controller is configurable to determine a temperature-compensated concentration of the analyte based on the actual measured absorbance and real-time temperature.

Although the temperature-compensated concentration of analyte generated by controller 2820 is disclosed above with respect to the optical absorbance measurement device 6050 and temperature sensor 6080 used in conjunction with a microfluidic manifold analysis support structure 6000, other embodiments contemplated may not use a microfluidic manifold for mixing the reagent and supernatant/filtrate (agricultural sample fluid). The agricultural sample fluid may instead be processed through non-microfluidic devices (e.g., pumps, valves, flow conduits, etc.) fluidly coupled together via tubing/piping and used in conjunction with the measurement device 6050, temperature sensor 6080, and controller 2820 disclosed to obtain the same functionality and temperature-compensated analyte concentration results albeit in a less compact form factor. Preferably in such conventional flow systems, the temperature of the sample fluid should preferably still be measured upstream of the measurement device proximate to the inlet of the device for accurate temperature and analyte measurements. Examples of non-microfluidic slurry and agricultural sample fluid systems and devices which may be used include those disclosed in commonly-owned International Patent Applications PCT/IB2019/055862 and PCT/IB2021/052872.

### EXAMPLES

The following are non-limiting examples.

Example 1 - A microfluidic manifold for analyzing an agricultural sample comprising: a substrate comprising a plurality of microfluidic devices fluidly coupled together by microchannels configured to convey a sample fluid derived from the agricultural sample; a measurement device mounted to the substrate, the measurement device configured to measure an absorbance value associated with an analyte in the sample fluid; a temperature sensor configured to measure a real-time temperature of the sample fluid; and a programmable controller operably coupled to the measurement device and temperature sensor, the controller being configured to determine a temperature-compensated concentration of the analyte based on the measured absorbance value and real-time temperature.

Example 2 - the microfluidic manifold according to Example 1, wherein the temperature sensor is embedded internally in the substrate proximate to the sample fluid.

Example 3 - the microfluidic manifold according to Example 2, wherein the temperature sensor is mounted in a measurement bore formed through the substrate from a first outer surface of the substrate.

Example 4 - the microfluidic manifold according to 3, wherein the microfluidic device includes at least one micropump, the temperature sensor being configured to measure the real-time temperature of the sample fluid in the at least one micropump.

Example 5 - the microfluidic manifold according to Example 4, wherein the measurement bore comprises a closed terminal end separated from the micropump by a partition wall formed from the substrate.

Example 6 - the microfluidic manifold according to Examples 4 or 5, wherein the at least one micropump is located upstream of and proximate to the measurement device.

Example 7 - the microfluidic manifold according to Example 7, wherein the at least one micropump is fluidly coupled to the measurement device through a microvalve configured to control flow of the sample fluid between the at least one micropump and the measurement device.

Example 8 - the microfluidic manifold according to Example 3, wherein the measurement bore and temperature sensor are configured and arranged to measure the real-time temperature of the sample fluid upstream of the measurement device.

Example 9 - the microfluidic manifold according to any one of Examples 1-8, wherein the temperature sensor is a thermistor or a thermocouple.

Example 10 - the microfluidic manifold according to any one of Examples 1-9, wherein the controller is configured to receive the absorbance value from the measurement device and correlate the absorbance value to the concentration of analyte using a preprogrammed base calibration curve.

Example 11 - the microfluidic manifold according to Example 10, wherein the controller is configured to: receive the real-time temperature of the sample fluid from the temperature sensor; and automatically adjust the base calibration curve based on the real-time temperature.

Example 12 - the microfluidic manifold according to Example 10, wherein the controller uses a plurality of preprogrammed temperature compensation curves to adjust the base calibration curve based on the real-time temperature of the sample fluid received from the temperature sensor.

Example 13 - the microfluidic manifold according to Example 12, wherein the temperature compensation curves provide a variance of absorbance versus a range of temperatures for plural calibration standard fluids each having a different know concentration of the analyte.

Example 14 - the microfluidic manifold according to any one of Examples 1-13, wherein the measurement device is an optical absorbance measurement device.

Example 15 - the microfluidic manifold according to any one of Examples 1-14, wherein the measurement device is mounted in a mounting aperture extending through and between opposite first and second major sides of the substrate.

Example 16 - the microfluidic manifold according to Example 15, wherein the measurement device includes a transmitter printed circuit board mounted adjacent to the first major side and an opposing detector printed circuit board mounted adjacent to the second major side.

Example 17 - the microfluidic manifold according to any one of Examples 14-16, wherein the measurement device includes an elongated measurement passageway formed between a pair of transparent windows, an inlet fluidly coupled to the measurement passageway and configured to receive the sample fluid from one of the microchannels, and an outlet fluidly coupled to the measurement passageway and configured to receive the sample fluid therefrom and return the sample fluid to another one of the microchannels.

Example 18 - the microfluidic manifold according to Example 17, wherein the measurement passageway is vertically oriented and operable to carry entrained gas in the sample fluid out of the measurement passageway.

Example 19 - the microfluidic manifold according to Example 18, wherein the sample fluid flows upwards in the measurement passageway.

Example 20 - the microfluidic manifold according to any one of Examples 1-19, wherein the measurement device is threadably coupled to the substrate by fasteners.

Example 21 - the microfluidic manifold according to any one of Examples 1-20, wherein the substrate is multi-layered comprised of a plurality of layers formed of polymeric material joined together.

Example 22 - the microfluidic manifold according to Example 21, wherein the microfluidic devices are embedded within the layers between opposing outer major surfaces of the substrate.

Example 23 - A method for analyzing an agricultural sample comprising: providing a substrate comprising a plurality of microfluidic devices fluidly coupled together by microchannels configured to convey a sample fluid derived from the agricultural sample; measuring an absorbance value associated with an analyte in the sample fluid with a measurement device; measuring a real-time temperature of the sample fluid with a temperature sensor; and determining with programmable controller a temperature-compensated concentration of the analyte based on the measured absorbance value and real-time temperature.

Example 24 - the method according to Example 23, wherein the controller receives the absorbance value from the measurement device and correlates the absorbance value to the concentration of analyte using a preprogrammed base calibration curve.

Example 25 - the method according to Example 24, wherein the controller receives the real-time temperature of the sample fluid from the temperature sensor and automatically adjusts the base calibration curve based on the real-time temperature.

Example 26 - the method according to Example 25, wherein the controller uses a plurality of preprogrammed temperature compensation curves to adjust the base calibration curve based on the real-time temperature of the sample fluid received from the temperature sensor.

Example 27 - the method according to Example 26, wherein the temperature compensation curves provide a variance of absorbance versus a range of temperatures for different calibration standard fluids each having a different know concentration of the analyte.

Example 28 - the method according to any one of Examples 23-27, wherein the measurement device is an optical absorbance measurement device.

Example 29 - the method according to any one of Examples 23-28, wherein the step of measuring the absorbance value associated with the analyte further comprises flowing the sample fluid vertically upwards through a measurement passageway of the measurement device.

Example 30 - A system for analyzing an agricultural sample comprising: a substrate comprising a plurality of flow control devices fluidly coupled together by flow conduits configured to convey a sample fluid derived from the agricultural sample; a measurement device configured to measure an absorbance value associated with an analyte in the sample fluid; a temperature sensor configured to measure a real-time temperature of the sample fluid; and a programmable controller operably coupled to the measurement device and temperature sensor, the controller being configured to determine a temperature-compensated concentration of the analyte based on the measured absorbance and real-time temperature.

## Claims

1. A microfluidic manifold for analyzing an agricultural sample comprising:
a substrate (6000) comprising a plurality of microfluidic devices fluidly coupled together by microchannels (4012) configured to convey a sample fluid derived from the agricultural sample;
a measurement device (6050) mounted to the substrate (6000), the measurement device (6050) configured to measure an absorbance value associated with an analyte in the sample fluid;
a temperature sensor (6080) configured to measure a real-time temperature of the sample fluid; and
a programmable controller (2820) operably coupled to the measurement device (6050) and temperature sensor (6080),
**characterized in that**
the programmable controller (2820) is configured to determine a temperature-compensated concentration of the analyte based on the measured absorbance value and real-time temperature.

2. The microfluidic manifold according to claim 1, wherein the temperature sensor (6080) is embedded internally in the substrate (6000) proximate to the sample fluid.

3. The microfluidic manifold according to claim 2, wherein the temperature sensor (6080) is mounted in a measurement bore (6082) formed through the substrate (6000) from a first outer surface of the substrate (6000).

4. The microfluidic manifold according to 3, wherein the microfluidic device includes at least one micropump (5015), the temperature sensor (6080) being configured to measure the real-time temperature of the sample fluid in the at least one micropump (5015).

5. The microfluidic manifold according to claim 4, wherein the measurement bore (6082) comprises a closed terminal end separated from the micropump (5015) by a partition wall formed from the substrate (6000).

6. The microfluidic manifold according to claims 4 or 5, wherein the at least one micropump (5015) is located upstream of and proximate to the measurement device (6050).

7. The microfluidic manifold according to claim 7, wherein the at least one micropump (5015) is fluidly coupled to the measurement device (6050) through a microvalve configured to control flow of the sample fluid between the at least one micropump (5015) and the measurement device (6050).

8. The microfluidic manifold according to claim 3, wherein the measurement bore (6082) and temperature sensor (6080) are configured and arranged to measure the real-time temperature of the sample fluid upstream of the measurement device (6050).

9. The microfluidic manifold according to any one of claims 1-8, wherein at least one of:
the temperature sensor (6080) is a thermistor or a thermocouple; and/or
the programmable controller (2820) is configured to receive the absorbance value from the measurement device (6050) and correlate the absorbance value to the concentration of analyte using a preprogrammed base calibration curve, optionally, wherein the programmable controller (2820) is configured to: receive the real-time temperature of the sample fluid from the temperature sensor (6080); and automatically adjust the base calibration curve based on the real-time temperature, and/or the programmable controller (2820) uses a plurality of preprogrammed temperature compensation curves to adjust the base calibration curve based on the real-time temperature of the sample fluid received from the temperature sensor (6080), and optionally wherein the temperature compensation curves provide a variance of absorbance versus a range of temperatures for plural calibration standard fluids each having a different know concentration of the analyte.

10. The microfluidic manifold according to any one of claims 1-9, wherein at least one of:
the measurement device (6050) is an optical absorbance measurement device (6050); and/or
the measurement device (6050) is mounted in a mounting aperture extending through and between opposite first and second major sides of the substrate (6000), optionally wherein the measurement device (6050) includes a transmitter printed circuit board mounted adjacent to the first major side and an opposing detector printed circuit board mounted adjacent to the second major side.

11. The microfluidic manifold according to claim 10, wherein the measurement device (6050) includes an elongated measurement passageway formed between a pair of transparent windows, an inlet fluidly coupled to the measurement passageway and configured to receive the sample fluid from one of the microchannels (4012), and an outlet fluidly coupled to the measurement passageway and configured to receive the sample fluid therefrom and return the sample fluid to another one of the microchannels (4012).

12. The microfluidic manifold (6000) according to claim 11, wherein the measurement passageway is vertically oriented and operable to carry entrained gas in the sample fluid out of the measurement passageway, optionally wherein the sample fluid flows upwards in the measurement passageway.

13. The microfluidic manifold according to any one of claims 1-12, wherein at least one of:
the measurement device (6050) is threadably coupled to the substrate (6000) by fasteners; and/or
the substrate (6000) is multi-layered comprised of a plurality of layers formed of polymeric material joined together, optionally the microfluidic devices are embedded within the layers between opposing outer major surfaces of the substrate (6000).

14. A method for analyzing an agricultural sample comprising:
providing a substrate (6000) comprising a plurality of microfluidic devices (6000) fluidly coupled together by microchannels (4012) configured to convey a sample fluid derived from the agricultural sample;
measuring an absorbance value associated with an analyte in the sample fluid with a measurement device (6050);
measuring a real-time temperature of the sample fluid with a temperature sensor (6080); **characterized by**
determining with programmable controller (2820) a temperature-compensated concentration of the analyte based on the measured absorbance value and real-time temperature.

15. The method according to claim 14, wherein the programmable controller (2820) receives the absorbance value from the measurement device (6050) and correlates the absorbance value to the concentration of analyte using a preprogrammed base calibration curve;
optionally, wherein the programmable controller (2820) receives the real-time temperature of the sample fluid from the temperature sensor (6080) and automatically adjusts the base calibration curve based on the real-time temperature;
optionally, wherein the programmable controller (2820) uses a plurality of preprogrammed temperature compensation curves to adjust the base calibration curve based on the real-time temperature of the sample fluid received from the temperature sensor (6080);
optionally, wherein the temperature compensation curves provide a variance of absorbance versus a range of temperatures for different calibration standard fluids each having a different know concentration of the analyte.

16. The method according to any one of claims 14 or 15, wherein at least one of:
the measurement device (6050) is an optical absorbance measurement device (6050); and/or
the step of measuring the absorbance value associated with the analyte further comprises flowing the sample fluid vertically upwards through a measurement passageway of the measurement device (6050).

## Patentansprüche

1. Mikrofluidischer Verteiler oder mikrofluidische Einrichtung zum Analysieren einer landwirtschaftlichen Probe mit:
einem Substrat (6000) mit mehreren fluidischen Geräten, die fluidisch über Mikrokanäle (4012) miteinander gekoppelt sind, die konfiguriert sind, um ein Probenfluid, welches von der landwirtschaftlichen Probe gewonnen ist, zu überbringen;
einem Messgerät (6050), das an dem Substrat (6000) montiert ist, wobei das Messgerät (6050) konfiguriert ist, um einen Absorptionswert zu messen, der einem Analyt in dem Probenfluid zugeordnet ist;
einem Temperatursensor (6080), der konfiguriert ist, um eine Echtzeit-Temperatur des Probenfluids zu messen; und
einem programmierbaren Controller (2820), der betrieblich mit dem Messgerät (6050) und dem Temperatursensor (6080) gekoppelt ist,
**dadurch gekennzeichnet, dass**
der programmierbare Controller (2820) konfiguriert ist, um eine temperaturkompensierte Konzentration des Analyts auf Basis des gemessenen Absorptionswerts und der Echtzeit-Temperatur zu ermitteln.

2. Mikrofluidischer Verteiler oder mikrofluidische Einrichtung nach Anspruch 1, wobei der Temperatursensor (6080) in der Nähe des Probenfluid innen in das Substrat (6000) eingebettet ist.

3. Mikrofluidischer Verteiler oder mikrofluidische Einrichtung nach Anspruch 2, wobei der Temperatursensor (6080) in einer Messbohrung (6082) montiert ist, die von einer ersten äußeren Oberfläche des Substrats (6000) durch das Substrat (6000) ausgebildet ist.

4. Mikrofluidischer Verteiler oder mikrofluidische Einrichtung nach Anspruch 3, wobei das mikrofluidische Gerät mindestens eine Mikropumpe (5015) aufweist, wobei der Temperatursensor (6080) konfiguriert ist, um die Echtzeit-Temperatur des Probenfluids in der mindestens einen Mikropumpe (5015) zu messen.

5. Mikrofluidischer Verteiler oder mikrofluidische Einrichtung nach Anspruch 4, wobei die Messbohrung (6082) ein geschlossenes Ende oder Terminal-Ende aufweist, welches von der Mikropumpe (5015) durch eine Trennwand getrennt ist, die von dem Substrat (6000) ausgebildet ist.

6. Mikrofluidischer Verteiler oder mikrofluidische Einrichtung nach Anspruch 4 oder 5, wobei die mindestens eine Mikropumpe (5015) stromaufwärts von dem Messgerät (6050) und in der Nähe desselben angeordnet ist.

7. Mikrofluidischer Verteiler oder mikrofluidische Einrichtung nach Anspruch 7, wobei die mindestens eine Mikropumpe (5015) fluidisch mit dem Messgerät (6050) über ein Mikroventil gekoppelt ist, welches konfiguriert ist, um den Fluss des Probenfluids zwischen der mindestens einen Mikropumpe (5015) und dem Messgerät (6050) zu steuern.

8. Mikrofluidischer Verteiler oder mikrofluidische Einrichtung nach Anspruch 3, wobei die Messbohrung (6082) und der Temperatursensor (6080) konfiguriert sind und geeignet angeordnet sind, um die Echtzeit-Temperatur des Probenfluids stromaufwärts des Messgeräts (6050) zu messen.

9. Mikrofluidischer Verteiler oder mikrofluidische Einrichtung nach einem der Ansprüche 1 bis 8, wobei mindestens eine der folgenden Bedingungen gilt:
der Temperatursensor (6080) ist ein Thermistor, ein NTC-Widerstand, ein temperaturgesteuerter Widerstand, ein wärmeabhängiger Widerstand oder ein Thermokoppler; und/oder
der programmierbare Controller (2820) ist konfiguriert, um den Absorptionswert von dem Messgerät (6050) zu empfangen und den Absorptionswert mit der Konzentration des Analyts zu korrelieren unter Verwendung einer vorprogrammierten Basis-Kalibrierkurve, wobei vorzugsweise der programmierbare Controller (2820) konfiguriert ist um: die Echtzeit-Temperatur des Probenfluids von dem Temperatursensor (6080) zu empfangen; und automatisch die Basis-Kalibrierkurve anzupassen auf Basis der Echtzeit-Temperatur, und/oder der programmierbare Controller (2820) verwendet mehrere vorprogrammierte Temperatur-Kompensationskurven zur Anpassung der Basis-Kalibrierkurve auf Basis einer Echtzeit-Temperatur des Probenfluids, die von dem Temperatursensor (6080) empfangen wird, und wobei vorzugsweise die Temperatur-Kompensationskurven eine Varianz der Absorption gegenüber einem Bereich der Temperaturen für mehrere Kalibrier-Standardfluide, die jeweils unterschiedliche bekannte Konzentrationen des Analyts haben, bereitstellen.

10. Mikrofluidischer Verteiler oder mikrofluidische Einrichtung nach einem der Ansprüche 1 bis 9, wobei mindestens eine der folgenden Bedingungen gilt:
das Messgerät (6050) ist ein optisches Absorptions-Messgerät (6050); und/oder
das Messgerät (6050) ist in einer Montageöffnung montiert, die sich durch und zwischen gegenüberliegende(n) erste(n) und zweite(n) Hauptseiten des Substrats (6000) erstreckt, wobei vorzugsweise das Messgerät (6050) ein Transmitter-Printed-Circuit-Board aufweist, welches benachbart der ersten Hauptseite montiert ist, und ein gegenüberliegendes Detector-Printed-Circuit-Board aufweist, welches benachbart der zweiten Hauptseite montiert ist.

11. Mikrofluidischer Verteiler oder mikrofluidische Einrichtung nach Anspruch 10, wobei das Messgerät (6050) eine längliche Mess-Durchtrittsbahn aufweist, die zwischen einem Paar von durchsichtigen oder durchscheinenden Fenstern gebildet ist, und einen Einlass aufweist, der fluidisch mit der Mess-Durchtrittsbahn gekoppelt ist und konfiguriert ist, um das Probenfluid von einem der Mikrokanäle (4012) zu empfangen, und einen Auslass aufweist, der fluidisch mit der Mess-Durchtrittsbahn gekoppelt ist und konfiguriert ist, um das Probenfluid hiervon zu empfangen und das Probenfluid zu einem anderen der Mikrokanäle (4012) zurückzuführen.

12. Mikrofluidischer Verteiler oder mikrofluidische Einrichtung (6000) nach Anspruch 11, wobei die Mess-Durchtrittsbahn vertikal orientiert ist und betrieben werden kann, um in dem Probenfluid enthaltenes Gas aus der Mess-Durchtrittsbahn zu tragen, wobei vorzugsweise das Probenfluid in der Mess-Durchtrittsbahn aufwärts fließt oder strömt.

13. Mikrofluidischer Verteiler oder mikrofluidische Einrichtung nach einem der Ansprüche 1 bis 12, wobei mindestens eine der folgenden Bedingungen erfüllt ist:
das Messgerät (6050) ist mittels einer Verschraubung über Befestigungsmittel mit dem Substrat (6000) gekoppelt; und/oder
das Substrat (6000) weist mehrere Schichten auf mit mehreren Schichten aus Polymermaterial, die miteinander verbunden sind, wobei vorzugsweise die mikrofluidischen Geräte in die Schichten eingebettet sind zwischen gegenüberliegenden Haupt-Oberflächen des Substrats (6000).

14. Verfahren zum Analysieren einer landwirtschaftlichen Probe mit:
einem Bereitstellen eines Substrats (6000) mit mehreren mikrofluidischen Geräten (6000), die fluidisch miteinander über Mikrokanäle (4012) gekoppelt sind, die konfiguriert sind, um ein Probenfluid, welches von der landwirtschaftlichen Probe gewonnen wird, zu überbringen;
einem Messen eines Absorptionswerts, der einem Analyt in dem Probenfluid zugeordnet ist, mittels eines Messgeräts (6050);
einem Messen einer Echtzeit-Temperatur des Probenfluids mit einem Temperatursensor (6080);
**gekennzeichnet durch**
ein Ermitteln einer temperatur-kompensierten Konzentration des Analyts auf Basis des gemessenen Absorptionswerts und der Echtzeit-Temperatur mittels eines programmierbaren Controllers (2820).

15. Verfahren nach Anspruch 14, wobei der programmierbare Controller (2820) den Absorptionswert von dem Messgerät (6050) empfängt und den Absorptionswert mit der Konzentration des Analyts unter Verwendung einer vorprogrammierten Basis-Kalibrierkurve korreliert;
wobei vorzugsweise der programmierbare Controller (2820) die Echtzeit-Temperatur des Probenfluids von dem Temperatursensor (6080) empfängt und automatisch die Basis-Kalibrierkurve auf Basis der Echtzeit-Temperatur anpasst;
wobei vorzugsweise der programmierbare Controller (2820) mehrere vorprogrammierte Temperatur-Kompensationskurven verwendet zur Anpassung der Basis-Kalibrierkurve auf Basis der Echtzeit-Temperatur des Probenfluids, welche von dem Temperatursensor (6080) empfangen wird;
wobei vorzugsweise die Temperatur-Kompensationskurven eine Varianz der Absorption gegenüber einem Bereich von Temperaturen für unterschiedliche Kalibrier-Standardfluide, die jeweils unterschiedliche bekannte Konzentrationen des Analyts aufweisen, bereitstellen.

16. Verfahren nach einem der Ansprüche 14 oder 15, wobei mindestens eine der folgenden Bedingungen erfüllt ist:
das Messgerät (6050) ist ein optisches Absorptions-Messgerät (6050); und/oder
der Schritt des Messens des Absorptionswerts, der dem Analyt zugeordnet ist, weist des Weiteren ein Strömen des Probenfluids vertikal aufwärts durch eine Mess-Durchtrittsbahn des Messgeräts (6050) auf.

## Revendications

1. Collecteur micro-fluidique destiné à analyser un échantillon agricole comprenant :
un substrat (6000) comprenant une pluralité de dispositifs micro-fluidiques couplés entre eux hydrauliquement par des micro-canaux (4012) configurés de manière à transférer un échantillon de fluide dérivé de l'échantillon agricole ;
un dispositif de mesure (6050) monté sur le substrat (6000), le dispositif de mesure (6050) étant configuré de manière à mesurer une valeur de facteur d'absorption associée à un analyte dans l'échantillon de fluide ;
un capteur de température (6080) configuré de manière à mesurer une température de l'échantillon de fluide en temps réel ; et
une unité de traitement programmable (2820) couplée de manière opérationnelle au dispositif de mesure (6050) et au capteur de température (6080),
**caractérisé en ce que**
l'unité de traitement programmable (2820) est configurée de manière à déterminer une concentration en analyte corrigée en fonction de la température sur la base de la valeur de facteur d'absorption et de la température en temps réel mesurées.

2. Collecteur micro-fluidique selon la revendication 1, dans lequel le capteur de température (6080) est intégré à l'intérieur du substrat (6000) à proximité de l'échantillon de fluide.

3. Collecteur micro-fluidique selon la revendication 2, dans lequel le capteur de température (6080) est monté dans un puits de mesure (6082) formé à travers le substrat (6000) à partir d'une première surface externe du substrat (6000).

4. Collecteur micro-fluidique selon la revendication 3, dans lequel le dispositif micro-fluidique comporte au moins une micro-pompe (5015), le capteur de température (6080) étant configuré de manière à mesurer la température de l'échantillon de fluide en temps réel dans la au moins une micro-pompe (5015).

5. Collecteur micro-fluidique selon la revendication 4, dans lequel le puits de mesure (6082) comporte une extrémité terminale fermée, séparée de la micro-pompe (5015) par une paroi de séparation formée à partir du substrat (6000).

6. Collecteur micro-fluidique selon les revendications 4 ou 5, dans lequel la au moins une micro-pompe (5015) est située en amont et à proximité du dispositif de mesure (6050).

7. Collecteur micro-fluidique selon la revendication 6, dans lequel la au moins une micro-pompe (5015) est couplée hydrauliquement au dispositif de mesure (6050) par l'intermédiaire d'une micro-vanne configurée de manière à commander l'écoulement de l'échantillon de fluide entre la au moins une micro-pompe (5015) et le dispositif de mesure (6050).

8. Collecteur micro-fluidique selon la revendication 3, dans lequel le puits de mesure (6082) et le capteur de température (6080) sont configurés et agencés de manière à mesurer la température de l'échantillon de fluide en temps réel, en amont du dispositif de mesure (6050).

9. Collecteur micro-fluidique selon l'une quelconque des revendications 1 à 8, dans lequel au moins une condition est réunie parmi les suivantes :
le capteur de température (6080) est une thermistance ou un thermocouple ; et/ou
l'unité de traitement programmable (2820) est configurée de manière à recevoir la valeur de facteur d'absorption à partir du dispositif de mesure (6050) et à corréler la valeur de facteur d'absorption avec la concentration en analyte en utilisant une courbe d'étalonnage de base préprogrammée, dans lequel, en variante, l'unité de traitement programmable (2820) est configurée de manière à : recevoir la température de l'échantillon de fluide en temps réel à partir du capteur de température (6080) ; et à ajuster automatiquement la courbe d'étalonnage de base en fonction de la température en temps réel, et/ou l'unité de traitement programmable (2820) utilise une pluralité de courbes de correction en fonction de la température préprogrammées afin d'ajuster la courbe d'étalonnage de base en fonction de la température de l'échantillon de fluide en temps réel, reçue à partir du capteur de température (6080) et dans lequel, en variante, les courbes de correction en fonction de la température fournissent une variation d'absorption en fonction d'une plage de températures pour plusieurs fluides d'étalonnage standard, chacun présentant une concentration en analyte différente connue.

10. Collecteur micro-fluidique selon l'une quelconque des revendications 1 à 9, dans lequel au moins une condition est réunie parmi les suivantes :
le dispositif de mesure (6050) est un dispositif de mesure d'absorption optique (6050) ; et/ou
le dispositif de mesure (6050) est monté sur une ouverture de montage s'étendant à travers des première et seconde faces principales opposées du substrat (6000) et entre ces dernières, dans lequel, en variante, le dispositif de mesure (6050) comporte une carte de circuit imprimé d'émetteur, montée de manière adjacente à la première face principale et une carte de circuit imprimé de détecteur opposée, montée de manière adjacente à la seconde face principale.

11. Collecteur micro-fluidique selon la revendication 10, dans lequel le dispositif de mesure (6050) comporte une voie de passage de mesure allongée formée entre une paire de fenêtres transparentes, une entrée couplée hydrauliquement à la voie de passage de mesure et configurée de manière à recevoir un échantillon de fluide à partir de l'un des micro-canaux (4012) et une sortie couplée hydrauliquement à la voie de passage de mesure et configurée de manière à recevoir l'échantillon de fluide à partir de celle-ci et à renvoyer l'échantillon de fluide vers un autre des micro-canaux (4012).

12. Collecteur micro-fluidique (6000) selon la revendication 11, dans lequel la voie de passage de mesure est orientée verticalement et peut servir à acheminer les gaz entraînés dans l'échantillon de fluide hors de la voie de passage de mesure, dans lequel, en variante, l'échantillon de fluide s'écoule vers le haut dans la voie de passage de mesure.

13. Collecteur micro-fluidique selon l'une quelconque des revendications 1 à 12, dans lequel au moins une condition est réunie parmi les suivantes :
le dispositif de mesure (6050) est couplé par vissage au substrat (6000) par des éléments de fixation ; et/ou
le substrat (6000) est multicouche, constitué par une pluralité de couches formées de matériau polymère reliées entre elles, en variante, les dispositifs micro-fluidiques sont noyées à l'intérieur des couches entre les surfaces principales externes opposées du substrat (6000).

14. Procédé d'analyse d'un échantillon agricole comprenant :
la fourniture d'un substrat (6000) comprenant une pluralité de dispositifs micro-fluidiques (6000) couplés hydrauliquement entre eux par des micro-canaux (4012) configurés de manière à convoyer un échantillon de fluide dérivé de l'échantillon agricole ;
la mesure d'une valeur de facteur d'absorption associée à un analyte dans l'échantillon de fluide avec un dispositif de mesure (6050) ;
la mesure d'une température de l'échantillon de fluide en temps réel avec un capteur de température (6080) ;
**caractérisé par**
la détermination, avec l'unité de traitement programmable (2820), d'une concentration en analyte corrigée en fonction de la température sur la base de la valeur de facteur d'absorption et de la température en temps réel mesurées.

15. Procédé selon la revendication 14, dans lequel l'unité de traitement programmable (2820) reçoit la valeur de facteur d'absorption à partir du dispositif de mesure (6050) et réalise la corrélation entre la valeur de facteur d'absorption et la concentration en analyte en utilisant une courbe d'étalonnage de base préprogrammée ;
dans lequel, en variante, l'unité de traitement programmable (2820) reçoit la température de l'échantillon de fluide en temps réel à partir du capteur de température (6080) et ajuste automatiquement la courbe d'étalonnage de base en fonction de la température en temps réel ;
dans lequel, en variante, l'unité de traitement programmable (2820) utilise une pluralité de courbes de correction en fonction de la température préprogrammées afin d'ajuster la courbe d'étalonnage de base en fonction de la température de l'échantillon de fluide en temps réel, reçue à partir du capteur de température (6080) ;
dans lequel, en variante, les courbes de correction en fonction de la température fournissent une variation de facteur d'absorption en fonction d'une plage de températures pour plusieurs fluides d'étalonnage standard, chacun présentant une concentration en analyte différente connue.

16. Procédé selon l'une quelconque des revendications 14 ou 15, dans lequel au moins une condition est réunie parmi les suivantes :
le dispositif de mesure (6050) est un dispositif de mesure de facteur d'absorption optique (6050) ; et/ou
l'étape de mesure de la valeur de facteur d'absorption associée à l'analyte comprend, en outre, le transfert de l'échantillon de fluide verticalement vers le haut par l'intermédiaire d'une voie de passage de mesure du dispositif de mesure (6050).
